(19) Europäisches Patentamt
European Patent Office
Office européen des brevets

(11) EP 4 262 472 B1

(12) **EUROPEAN PATENT SPECIFICATION**

(45) Date of publication and mention of the grant of the patent:
**15.07.2026 Bulletin 2026/29**

(21) Application number: **21831046.4**

(22) Date of filing: **16.12.2021**

(51) International Patent Classification (IPC):
***A45D 40/26*** $^{(2006.01)}$ ***A45D 34/04*** $^{(2006.01)}$
***A61K 8/02*** $^{(2006.01)}$ ***A61K 8/44*** $^{(2006.01)}$
***A61Q 1/10*** $^{(2006.01)}$

(52) Cooperative Patent Classification (CPC):
**A61K 8/0295; A45D 34/042; A45D 40/262; A61K 8/447; A61Q 1/10;** A46B 2200/1053

(86) International application number:
**PCT/EP2021/086086**

(87) International publication number:
**WO 2022/129277 (23.06.2022 Gazette 2022/25)**

(54) **KERATIN FIBERS SHAPING TOOL, AND ASSOCIATED KITS AND METHOD**

WERKZEUG ZUR FORMUNG VON KERATINFASERN SOWIE ZUGEHÖRIGE KITS UND VERFAHREN

OUTIL DE MISE EN FORME DE FIBRES DE KÉRATINE ET KITS ET PROCÉDÉ ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2020 FR 2013387**
**16.12.2020 FR 2013377**

(43) Date of publication of application:
**25.10.2023 Bulletin 2023/43**

(73) Proprietor: **L'OREAL**
**75008 Paris (FR)**

(72) Inventors:
- **ILEKTI, Philippe**
  **94152 CHEVILLY LA RUE (FR)**
- **RIZZO, Karl-Joseph**
  **94152 CHEVILLY LA RUE (FR)**
- **PLOS, Grégory**
  **94152 CHEVILLY LA RUE (FR)**

(74) Representative: **Lavoix**
**2, place d'Estienne d'Orves**
**75441 Paris Cedex 09 (FR)**

(56) References cited:
**WO-A1-2017/115825** **FR-A1- 2 936 418**
**FR-A1- 3 026 945** **JP-A- 2005 192 996**
**JP-A- S61 199 807** **KR-B1- 101 823 336**
**KR-B1- 101 823 401** **US-A1- 2005 217 691**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 262 472 B1

## Description

The present invention relates to a tool for shaping keratin fibers.

Generally, compositions dedicated to makeup for keratin fibers, such as eyelashes or eyebrows, aim to densify the thickness and the visual perception of the eyelashes or eyebrows and ultimately the look. Documents JP 2005 192996 and JP S61199807 disclose examples of mascara applicator to apply cosmetic compositions on the keratin fibers.

However, this use is not satisfactory because the coverage is ephemeral, and the curvature of the eyelashes returns to its natural state, once the mascara makeup is removed.

Thus, another property that is especially sought by consumers is to obtain a significant curling of said keratin fibers that is persistent to successive makeup removals.

The term "curling" means a difference in angle between the chord before application, passing through the base of the keratin fiber, preferably of the eyelash, and by the tip of the chord after application, by at least 10°, preferably by at least 15°, preferably by at least 20°.

In addition, "persistent curling" means a curling that persists even after makeup removal. This persistent curling can last at least a few hours, preferably at least a few days.

For this purpose, it is also known to shape the eyelashes with an eye-curler that provides such a persistent curling. The eye-curler generally consists of a clamp that can be actuated manually, the clamp clamping the eyelashes in order to curl them.

However, the eye-curler does not provide full satisfaction. In particular, handling the eye-curler is delicate for the user. In addition, the handling is accompanied by a repeated clamping of the eyelashes or maintained in a prolonged manner which can damage the eyelashes. Such damaged eyelashes break or are pulled out.

A purpose of the invention is therefore to provide a solution that makes it possible to provide a curling of keratin fibers, in particular the eyelashes, that is simple to use, without damage for the fibers and as durable as possible.

An additional purpose is that the solution makes it possible to obtain a persistent curling of the fibers.

Another purpose is to ensure good separation of the fibers during the shaping.

For this purpose, the object of the invention is a shaping tool according to claim 1.

The tool according to the invention is indeed entirely adapted to the shaping of keratin fibers in a way that is easy to use and without damage for the fibers, without the constraints associated with usual applicators intended to apply a mascara composition. The shape of the usual applicators intended for applying a mascara composition is indeed constrained by the usage thereof, in particular at the applicator that has to cooperate with a squeezing device able to control the volume of the composition loaded onto the applicator. The tool of the invention is thus released from these constraints to provide an optimal shape for shaping fibers.

In particular, the particular shape of this tool, in particular at the distal edge of its shaping member, provides a useful zone over the entire length of the shaping member and minimizes the discomfort at contact with the corner of the eye, when the fibers are eyelashes.

The shaping tool according to the invention may be according to any one of claims 2 to 11.

The invention also relates to a cosmetic application kit according to any one of claim 12, 13 or 14.

The invention further relates to a method for shaping keratin fibers according to claim 15.

The present invention also relates to a makeup method for keratin fibers according to claim 16 and an associated kit, allowing for a persistent curling of said fibers.

Generally, compositions dedicated to makeup for keratin fibers, such as eyelashes or eyebrows, aim to densify the thickness and the visual perception of the eyelashes or eyebrows and *ultimately* the look. But another property that is especially sought by consumers is to obtain a significant curling of said keratin fibers that is persistent to successive makeup removals.

In a salon, professionals use a makeup method inspired by permanent hair techniques that consists, firstly, in applying on the eyelashes a reducing composition comprising a reducing agent (for example ammonium thioglycolate and the derivatives thereof or thiol compounds) that break the disulfide bridges of the keratin of the eyelashes then, secondly, in applying an oxidizing composition comprising at least one oxidizing agent such as hydrogen peroxide to reestablish the disulfide bridges of the keratin of the eyelashes, shape the eyelashes and obtain a durable curling. This method has the disadvantages of being constraining, uncomfortable and of not giving the consumer the possibility of easily applying this gesture themselves at home.

Patent applications KR101823336 and KR101823401 propose a method for the semi-permanent curling of eyelashes comprising the application on the eyelashes of a mascara comprising at least one reducing agent such as cysteine, cysteamine, thiol compounds, sodium bisulfite, sodium metabisulfite, sodium hydrosulfite or superoxide dismutase, and at least one antioxidant such as a compound with a phosphoric acid base or a compound with a zinc, gold, tin or silver base, followed by a washing and a drying of the treated eyelashes. This technique of semi-permanent curling of the eyelashes is not fully satisfactory on the significant curling effect obtained and on the persistence of the effect after makeup removal.

A need therefore subsists for a makeup method for keratin fibers, in particular eyelashes, that is easy via its gesture to implement at home for the consumer, and that produces an effective curling of said fibers while still being persistent, without the constraints of the known methods of the prior art mentioned hereinabove such as the use of an oxidizing agent.

The need also exists for a makeup method for keratin fibers, in particular eyelashes, that has a significant and persistent curling effect after makeup removal.

Unexpectedly, the inventors have observed during their research that it is possible to achieve this objective with a makeup method comprising a first application step on said keratin fibers of a composition comprising:

- at least 30% by weight of water with respect to the total weight of the composition, forming an aqueous phase,
- at least 1% by weight of with respect to the total weight of the composition of cysteine and/or one of the salts thereof,
- a lamellar phase Lβ formed by a surfactant system structuring the aqueous phase, and
  said composition having a pH greater than 8.0,

Then a second step of shaping said fibers by application on the latter of a shaping tool, said shaping tool comprising a gripping member extending along a longitudinal axis X, and a shaping member comprising a base body and ribs,

the base body being integral with the gripping member, and having two lateral edges, and extending from the gripping member to a free distal edge opposite the gripping member, the free distal edge joining the two lateral edges to one another,

each rib extending protruding from the base body in a respective elevation direction Z, the ribs also extending parallel to one another and to a transverse axis Y and defining grooves between them.

The shaping tool is also called in the present application "tool according to the invention". Preferably, in the tool according to the invention, each rib has a constant height along the transverse axis Y between the two lateral edges of the base body, the free distal edge of the base body extending parallel to the transverse axis Y.

Indeed, the application of such a composition on the keratin fibers, in particularly the eyelashes, followed by the shaping of the fibers by the tool according to the invention, makes it possible to curl said fibers in a persistent manner, and this, without using an oxidizing agent. Such a method is easy to implement at home for the consumer. The curling of the keratin fibers obtained is significant, and it persists after makeup removal.

Thus, an object of the present invention is a makeup method for keratin fibers, in particular the eyelashes, comprising a first step of applying on said keratin fibers a composition comprising:

- at least 30% by weight of water with respect to the total weight of the composition, forming an aqueous phase,
- at least 1% by weight of with respect to the total weight of the composition of cysteine and/or one of the salts thereof,
- a lamellar phase Lβ formed by a surfactant system structuring the aqueous phase, and

said composition having a pH greater than 8.0,
then a second step of shaping said fibers by application on the latter of a tool according to the invention.

Preferably, such a method uses no oxidizing agent.

In the framework of the present invention, the term "keratin fibers" means in particular the eyelashes and the eyebrows.

"Physiologically acceptable" means being compatible with the skin and the appendages thereof, having a pleasant color and touch and that does not generate any unacceptable discomfort (tickling, tightness) that might discourage the consumer from using this composition.

The method according to the invention making use of the described shaping tool can include one or more of the following features, taken alone or in any technically possible combination:

- the composition comprises at least 2% by weight with respect to the total weight of the composition of cysteine and/or one of the salts thereof, preferably at least 3% by weight, preferably from 1.5 to 10% by weight, preferably from 2% to 7% by weight; and/or the pH of the composition is greater than or equal 8.5, and preferably comprised between 8.5 and 10.0;

- the composition comprises a lamellar phase Lβ formed by a surfactant system structuring the aqueous phase, said surfactant system being present in a content greater than or equal to 9% by weight with respect to the total weight of the composition and comprising at least one anionic surfactant having an HLB greater than or equal to 8 at 25°C, and at

least one nonionic surfactant with an HLB less than 8 at 25°C;

- the anionic surfactant having an HLB greater than or equal to 8 at 25°C is chosen from fatty acid salts, acyl glutamic acids and the salts thereof, and alkyl phosphates and the salts thereof;

- the nonionic surfactant with a HLB less than 8 at 25°C is chosen from:

  fatty alcohols in $C_{14}$-$C_{22}$, preferably in $C_{16}$-$C_{18}$, such as cetyl alcohol, stearyl alcohol or cetearylic alcohol;
  esters and ethers of (poly)oxyalkylene oses;
  fatty acid esters, in particular in $C_8$-$C_{24}$, and preferably in $C_{12}$-$C_{20}$ or in $C_{16}$-$C_{22}$, and (poly)glycerol, preferably comprising one or two glycerol groups, preferably said fatty acid comprising a linear or branched, saturated or unsaturated $C_{12}$-$C_{20}$ alkyl chain, preferably linear and saturated; preferably said fatty acid ester and (poly) glycerol is chosen from glycerol stearate, glyceryl laurate, glyceryl myristate, preferably is glycerol stearate;
  (poly)oxyalkylenated alcohols comprising a C8-C24 fatty alcohol ether and polyethylene glycol, said ether comprising from 1 to 6, better between 2 and 6, ethylene glycol units;
  and mixtures thereof;

- the surfactant system is such that:

  * the anionic surfactant having an HLB greater than or equal to 8 at 25°C is stearic acid, palmitic acid and/or myristic acid neutralized with an organic base, or the anionic surfactant with an HLB greater than or equal to 8 at 25°C is a cetyl phosphate salt, preferably potassium cetyl phosphate; and/or
  * the nonionic surfactant with an HLB less than 8 at 25°C is chosen from fatty alcohols in $C_{14}$-$C_{22}$, preferably in $C_{16}$-$C_{18}$, such as cetyl alcohol, stearyl alcohol or cetearylic alcohol, and

  preferably the concentration in anionic surfactant having an HLB greater than or equal to 8 at 25°C, is greater than or equal to 4% by weight, preferably between 5% and 30% by weight and even more preferably between 5% and 20% by weight with respect to the total weight of the composition, and/or the concentration in nonionic surfactant having an HLB less than or equal to 8 at 25°C, is greater than or equal to 4% by weight, preferably between 5% and 30% by weight and even more preferably between 5% and 20% by weight with respect to the total weight of the composition;

- the composition comprises an Lβ lamellar phase formed by a surfactant system structuring the aqueous phase, said surfactant system being present with a content greater than or equal to 15% by weight with respect to the total weight of the composition and comprising at least 5% by weight with respect to the total weight of the composition of a nonionic surfactant having an HLB greater than or equal 8 at 25°C, and at least 5% by weight with respect to the total weight of the composition of a nonionic surfactant having an HLB of less than 8 at 25°C;

- the surfactant system comprises a mixture of:

  at least one nonionic surfactant having an HLB of less than 8 at 25°C chosen from C16-24 ethoxylated fatty alcohols comprising 1 to 6 ethylene glycol units, such as ethoxylated stearyl alcohol with 2 oxyethylene units; and
  at least two nonionic surfactants having an HLB greater than or equal to 8 at 25°C which are a mixture of at least one C16-22 ester of fatty acids and of oxyethylenated glycerol ethers including from 10 to 250 oxyethylenated groups, such as polyethoxylated glyceryl stearate with 30 ethylene oxide groups, and at least one oxyethylene ether, including from 10 to 250 oxyethylene groups, of C12-18 fatty alcohols, such as oxyethylene ether of stearyl alcohol with 20 oxyethylene groups;

- the surfactant system comprises a mixture of at least one nonionic surfactant having an HLB of less than 8 at 25°C chosen from the fatty alcohols in C14-C22, preferably in C16-C18, such as cetyl alcohol, stearyl alcohol or cetearylic alcohol; and of at least one nonionic surfactant having an HLB greater than or equal to 8 at 25°C chosen from fatty acid esters in particularly C8-C24 acid, and preferably C16-C22 acid, and of oxyethylenated and/or oxypropylated glycerol ethers that can comprise from 10 to 250 oxyethylenated and/or oxypropylated groups, such as PEG-200 glyceryl monostearate or PEG-100 stearate.

The invention will be easier to understand after reading the following description, provided solely as an example and with reference to the appended drawings, wherein:

[Fig 1] [Fig 2] figures 1 and 2 are diagrammatical views of different sides of a first embodiment of a shaping tool

according to the invention; and

[Fig 3] figure 3 is a diagrammatical view similar to that of figure 2 of a second embodiment of a shaping tool according to the invention.

Shaping tool (tool according to the invention)

A first embodiment of a shaping tool 10A of keratin fibers is shown in figures 1 and 2. The keratin fibers are preferably eyelashes.

The shaping tool 10A is intended for shaping the fibers without applying any cosmetic composition on them.

In particular, the shaping tool 10A is preferably used after prior application on the keratin fibers of a cosmetic composition by another applicator separate from the shaping tool 10A, as described in more detail hereinafter.

The tool 10A comprises a gripping member 12 extending along a longitudinal axis X, and a shaping member 14.

The gripping member 12 is intended to be gripped by a user.

The gripping member 12 comprises in particular a rod 16, having for example a circular cross-section. Alternatively, the cross-section has another geometrical shape.

The cross-section of the rod 16 is perpendicular to the longitudinal direction X and is for example constant along the longitudinal axis X.

The cross-section of the rod 16 has a larger dimension, corresponding for example here to the diameter, comprised between 3 mm and 8 mm.

The gripping member 12 is monoblock in an embodiment.

The gripping element 12 is for example made of plastic, preferably of polyethylene.

The shaping member 14 is intended for coming into contact with the keratin fibers, in particular for the purpose of shaping them without applying any cosmetic composition on them.

As shown in figures 1 and 2, the shaping member 14 comprises a base body 18, and ribs 20 extending protruding from the base body 18, the ribs 20 extending also substantially parallel to one another and to a transverse axis Y by defining grooves 22 between them.

The shaping member 14 is for example monoblock. In particular, the base body 18 and the ribs 20 are from a single piece.

The shaping member 14 is for example made from a plastic material and is carried out via injection.

The shaping member 14 is preferably made from polyethylene or from thermoplastic elastomer, for example from SEBS.

Preferably, the shaping member 14 has at least one plane of symmetry perpendicular to the longitudinal axis X.

The base body 18 is integral with the gripping member 12.

Preferably, the base body 18 is removably integral with respect to the gripping member 12. The base body 18 is in particular able to be removably attached to the gripping member 12.

For this, the base body 18 comprises for example two cavities, not shown, on either side of the base body 18. Each cavity is able to cooperate with a free end of the gripping member 12, in such a way as to render the base body 18 integral with the gripping member 12.

For example, the end of the gripping member 12 is able to be snap-fit into each cavity.

It is this possible to provide a tool 10A that can be adapted to the user according to whether the user is left-handed or right-handed.

As shown in figure 1, the base body 18 has two lateral edges 24, and extends from the gripping member 12 to a free distal edge 26 opposite the gripping member 12, the free distal edge 26 joining the two lateral edges 24 to one another.

The term "free distal end" means that the shaping tool has a distal end formed by the free edge of the base body. In other words, the shaping tool stops longitudinally at the free distal edge of the base body. In other words, said free edge is not added against any other element of the shaping tool. Again in other words, said free edge is an edge in contact with a mass of air over its entire extent.

The distal edge 26 is in particular the edge of the base body 18 that is farthest from the gripping member 12.

More precisely, the base body 18 extends between a proximal edge 28 and the distal edge 26, by being delimited laterally by the lateral edges 24. The proximal edge 28 also joins the two lateral edges 24 to one another.

The distal edge 26 and the proximal edge 28 each delimit for example one of said cavities for receiving the gripping member 12.

In the example shown, the distal edge 26 extends parallel to the transverse axis Y.

Advantageously, the distal edge 26 of the base body 18 stops at the rib 30A that is farthest from the gripping member 12 (see figure 1).

"The distal edge stops at the rib" means that the base body 18 does not extend beyond said rib 30A.

In this example, the proximal edge 28 extends parallel to the transverse axis Y.

Advantageously, the proximal edge 28 of the base body 18 stops at one of the ribs 30B (see figure 1).

Thus, generally, the base body 18 stops at the two extreme ribs 30A, 30B, which are the farthest from each other.

The two lateral edges 24 extend in particular the gripping member 12.

Preferably, a separation W between the two lateral edges 24 of the base body 18 is substantially constant along the base body 18.

The separation W is for example taken with respect to the transverse axis Y.

The separation W is in particular substantially constant to the distal edge 26.

Thus, the separation W is in particular the same at the distal edge 26 and at the proximal edge 28.

The separation W is preferably comprised between 3.0 mm and 10.0 mm, advantageously between 7.0 mm and 9.0 mm.

Such a separation makes it possible to maintain the keratin fibers in the shaping member 14 and to impose the bending force over a sufficient length of the fibers. If the separation W exceeds these values, the application of the shaping member 14 can become uncomfortable la commissure of the eye, in the case where the fiber are eyelashes.

Each lateral edge 24 also has a respective lateral radius of curvature R greater than or equal to 2.0 cm, defined in projection in a plane parallel to the longitudinal axis X and to the transverse axis Y.

Preferably, as shown, each lateral edge 24 only has a single respective radius of curvature R, in projection in the plane parallel to the longitudinal axis X and to the transverse axis Y.

It is to be noted that figure 1 is not a strict projection on a plane parallel to the longitudinal axis X and to the transverse axis Y, the tool is shown by being slightly turned according to the axis X with respect to such a projection.

In particular, each lateral edge 24 does not have a point of inflection. Each lateral edge 24 retains the same concavity.

In the advantageous embodiment of figure 1, the smallest lateral radius of curvature R is comprised between 5.0 cm and 9.0 cm, preferably equal to 7 cm.

Such a lateral radius of curvature R makes it possible to grasp the entire fringe of keratin fibers. In addition, in the case where the fibers are eyelashes, this allows for an intuitive placement of the shaping member 14 with respect to the curvature of the eyelid.

Each groove 22, defined between two adjacent ribs 20, has a bottom 32 formed by the base body 18. "Two adjacent ribs" means two immediately neighboring ribs 20, i.e. that no other rib is inserted between them.

Advantageously, the base body 18 is solid. In particular, any cross-section of the base body 18, taken at the bottom 32 of one of the grooves 22, has a solid section delimited by a rear face 34, the lateral edges 24 and the bottom 32 of said groove 22.

As shown in figure 2, all the bottoms 32 of the grooves 22 follow a shape that has a main radius of curvature r, defined in projection in a plane parallel to the longitudinal axis X and perpendicular to the transverse axis Y.

More precisely, all the bottoms 32 of the grooves 22 follow a shape that has a single main radius of curvature r.

In other words, the two radii of curvature r and R are perpendicular to one another.

Said main radius of curvature r is greater than or equal to 2.0 cm.

In the embodiment shown in figure 2, the main radius of curvature r is such that all the bottoms 32 of the grooves 22 follow a planar shape.

In this case, the main radius of curvature r is for example greater than 10 times, preferably greater than 100 times, the length of the base body 18, taken along the longitudinal axis X. The length of the base body 18 is in particular defined between the distal edge 26 and the proximal edge 28.

The rear face 34 of the base body 18 joins the lateral edges 24, the distal edge 26 and the proximal edge 28.

The rear face 34 here has for example a concave shape in at least one cross-section the base body 18, or a rounded hollowed shape towards the inside. Such a shape makes it possible here to provide at the distal and proximal edges 26, 28 a sufficient thickness of the base body 18 to accommodate the cavities for receiving the end of the rod 16, by reducing even so the mass of the shaping member 14 at the central portion thereof.

The ribs 20 of the shaping member 14 shall now be described in more detail.

The ribs 20 are able to receive between them the keratin fibers, during the application of the tool against the fibers. The fibers are then in particular received in the grooves 22.

Each rib 20 extends protruding from the base body 18 in a respective elevation direction Z, in a cross-section perpendicular to the transverse axis Y.

The elevation direction Z is for example perpendicular to a plane passing through the two bottoms 32 adjacent to the rib 20.

In the example shown, where the main radius of curvature r is such that all the bottoms 32 of the grooves 22 follow a planar shape, the elevation directions Z are parallel to one another.

Each rib 20 extends protruding from the base body 18 in the respective elevation direction Z until a top 36.

The top 36 of each rib 20 is for example defined as the portion of the rib that is farthest from each adjacent groove 22 that surrounds the rib 20.

The respective elevation direction Z thus passes for example through said top 36.

The top 36 of each rib 20 therefore extends along the transverse axis Y over the entire width of the base body 18. The width of the top 36 of each rib 20, taken along the transverse axis Y, is substantially equal to the separation W of the base body 18.

The plane of symmetry of the shaping member 14 perpendicular to the longitudinal axis X passes for example through one of the elevation directions Z, namely through one of the tops 36.

Each rib 20 has a height D defined as the distance, presented on the elevation direction Z, between the bottom 32 of one of the adjacent grooves 22 and the top 36 of the rib 20.

The height D of each rib 20 is constant along the transverse axis Y between the two lateral edges 24 of the base body 18.

All the ribs 20 have for example the same height D.

The height D of each rib 20, taken along the elevation direction Z, is comprised between 1.0 mm and 4.0 mm, preferably comprised between 2.0 mm to 3.0 mm.

Such a height D makes it possible to maintain the fibers in the grooves 22, while still preventing contact with the eyelid, in the case where the fibers are eyelashes.

As shown in figure 2, each rib 20 has for example a shape with a triangular point, in a cross-section perpendicular to the transverse axis Y. More generally, each rib 20 has a shape with a triangular point, in a slot, or curved, in a cross-section perpendicular to the transverse axis Y.

The shape of the rib 20 is constant along the transverse axis Y.

For example, for each rib 20, a plane perpendicular to the longitudinal axis X and passing through the elevation direction Z of the rib 20 forms a plane of symmetry of the rib.

All the ribs 20 have for example the same shape.

In particular, the two extreme ribs 30A, 30B from which the base body 18 stops have the same shape as the other ribs 20.

In the example of figure 2, the difference P between the tops 36 of two adjacent ribs 20 is constant.

The difference P is advantageously comprised between 0.3 mm and 5.0 mm, preferably comprised between 1.0 mm and 2.0 mm, advantageously equal to 1.5 mm.

Such a difference P makes it possible to guarantee a good separation of the fibers, in particular eyelashes.

Preferably, the distance L between the tops 36 of the two extreme ribs 30A, 30B the farthest from one another is comprised between 1.0 cm and 5.0 cm, preferably comprised between 2.0 cm and 4.0 cm, advantageously comprised between 3.0 cm and 4.0 cm.

Alternatives of the embodiment hereinabove of the tool shall now be described.

In particular, the two extreme ribs 30A, 30B from which the base body 18 stops have shapes different from those of the other ribs 20.

The two extreme ribs 30A, 30B then have for example a shape that corresponds to a truncation of the shape of the other ribs 20, the base body 18 stopping at the truncated portion.

Alternatively, the lateral radii of curvature R are sufficiently large so that the lateral edges 24 of the base body 18 are parallel to the longitudinal axis X.

In this case, each lateral radius of curvature R is for example greater than 10 times, preferably greater than 100 times, the length of the base body 18, taken along the longitudinal axis X.

A second embodiment of a tool 10B according to the invention shall now be described in reference to figure 3. Only the differences with respect to the first embodiment 10B shall be described hereinafter.

In the second embodiment 10B, the main radius of curvature r is comprised between 1 cm and 10 cm, in such a way that all the bottoms 32 of the grooves 22 followed a curved shape.

The term "curved" means a non-flat shape and devoid of a point of inflection.

Preferably, the main radius of curvature r is comprised between 2 cm and 8 cm.

In this embodiment, the curvature center of the main radius of curvature r is disposed facing ribs 20, in projection in a plane parallel to the longitudinal axis X and perpendicular to the transverse axis Y.

In addition, each elevation direction Z is different by forming a non-zero angle with the other elevation directions. The elevation directions cross for example at the center of the main radius of curvature r.

In an alternative advantageously not shown of this embodiment 10B, the longitudinal axis X passing through the center of the cross-section of the rod 16 also passes through the center of curvature associated with the main radius of curvature r.

First embodiment of a kit

The invention also related to a first embodiment of a cosmetic application kit comprising at least the shaping tool 10A, 10B as described hereinabove, a cosmetic mascara composition and an applicator of the cosmetic composition, the applicator being separate from the shaping tool 10A, 10B.

The cosmetic mascara composition, or "mascara" is in particular a coating and/or care composition for keratin materials, in particular eyelashes or eyebrows.

Mascaras are essentially prepared according to two types of formulations: aqueous mascaras referred to as "cream mascaras", in the form of dispersion of waxes in the water, or anhydrous mascaras or with low water content, referred to as "waterproof" mascaras, in the form of dispersions of waxes in organic solvents.

Preferably, the mascara comprises a continuous aqueous or oily phase, and a dispersion of waxes.

An aqueous mascara typically comprises a continuous aqueous phase and a dispersion of waxes. Preferably, the waxes are present in a content of at least 10% by weight, preferably at least 15% by weight with respect to the total weight of the composition.

A waterproof mascara typically comprises a continuous oily phase and a dispersion of waxes. Preferably, the waxes are present in a content of at least 10% by weight, preferably at least 15% by weight with respect to the total weight of the composition.

The applicator of the first embodiment of the kit in particular comprises means, where applicable in relief, configured to engage with the fibers, so as to smooth and/or separate the eyelashes or the eyebrows. Such reliefs can include teeth, bristles or others.

The applicator is arranged to apply the composition on the eyelashes or the eyebrows, and can include for example a brush or a comb.

The brush can include a twisted core and bristles taken between the spires of the core, or be carried out yet in another manner.

The comb is for example made of a single piece by molding of plastic material.

In certain embodiments, the applicator is mounted at the end of a rod, which can be flexible, which can contribute to improving the comfort during application.

Second embodiment of a kit

The invention also relates to a second embodiment of an application kit comprising at least the shaping tool 10A, 10B as described hereinabove.

In addition, the second embodiment of the kit comprises a composition and an applicator of said composition which is separate from the shaping tool 10A, 10B.

The applicator of the second embodiment of the kit in particular comprises means, where applicable in relief, configured to engage with the fibers, so as to smooth and/or separate the eyelashes or the eyebrows. Such reliefs can include teeth, bristles or others.

The applicator of the second embodiment of the kit is arranged to apply the composition on the eyelashes or the eyebrows, and can include for example a brush or a comb.

The brush can include a twisted core and bristles taken between the spires of the core, or be carried out yet in another manner.

The comb is for example made of a single piece by molding of plastic material.

In certain embodiments, the applicator of the second embodiment of the kit is mounted at the end of a rod, which can be flexible, which can contribute to improving the comfort during application.

The composition of the second embodiment of the kit (composition according to the invention) comprises:

- at least 30% by weight of water with respect to the total weight of the composition, forming an aqueous phase,
- at least 1% by weight of with respect to the total weight of the composition of cysteine and/or one of the salts thereof,
- a lamellar phase L$\beta$ formed by a surfactant system structuring the aqueous phase, and

said composition having a pH greater than 8.0.

Unexpectedly, the inventors have observed during their research that it is possible to obtain a makeup method for keratin fibers, in particular eyelashes, that is easy via its gesture to implement at home for the consumer, and that produces an effective curling of said fibers while still being persistent.

Indeed, the application of such a composition on the keratin fibers, in particularly the eyelashes, followed by the shaping of the fibers by the shaping tool 10A, 10B, makes it possible to curl said fibers in a persistent manner, without using an oxidizing agent. Such a method is easy to implement at home for the consumer. The curling of the keratin fibers obtained is significant, and it persists after makeup removal.

The composition according to the invention (or reducing composition) shall now be described in more detail.

Composition according to the invention (or reducing composition)

According to the present invention, the composition comprises:

- at least 30% by weight of water with respect to the total weight of the composition, forming an aqueous phase,
- at least 1% by weight of with respect to the total weight of the composition of cysteine and/or one of the salts thereof,
- a lamellar phase Lβ formed by a surfactant system structuring the aqueous phase, and

said composition having a pH greater than 8.0.

The composition comprises at least 1% by weight of with respect to the total weight of the composition of cysteine and/or of one of the salts thereof. Of course, said salt is a cosmetically acceptable salt.

Preferably, the cysteine implemented is chosen from L-cysteine, D-cysteine or L,D-cysteine. L-cysteine or one of the salts thereof will be used more particularly.

The cysteine and/or one of the salts is present in the composition in a concentration of at least 1% by weight with respect to the total weight of the composition, preferably at least 2% by weight, preferably at least 3% by weight. Preferably, the content in cysteine and/or one of the salts ranges from 1.5% to 10% by weight of the total weight of the composition, preferably from 2% to 7% by weight.

The pH of the composition according to the invention is greater than 8.0, preferably greater than or equal to 8.5, and preferably comprised between 8.5 and 10.0.

The pH can be obtained and/or adjusted conventionally by adding alkaline agents, such as ammonia, monoethanolamine, diethanolamine, triethanolamine, triethanolamine, 2-amino 2-methyl 1-propanol, la 1,3-propanediamine, an alkali or ammonium carbonate or bicarbonate, for example sodium bicarbonate, an organic carbonate such as guanidine carbonate, or an alkali hydroxide such as soda or potash, all these compounds able of course to be take alone or in a mixture.

Preferably 2-amino 2-methyl 1-propanol or soda will be used.

It is also possible to use in addition to the alkalizing agent and/or acidifying agent a physiologically acceptable buffer such as a phosphate buffer, or a carbonate buffer.

Aqueous phase

The composition according to the invention comprises at least 30% by weight of water with respect to the total weight of the composition, forming an aqueous phase.

This aqueous phase is a continuous aqueous phase. The term "continuous" aqueous phase means that the composition has a conductivity, measured at 25°C, greater than 23 μS/cm (microSiemens/cm), with the conductivity being measured for example using a Mettler Toledo MPC227 conductometer and an Inlab730 conductivity measuring cell. The measuring cell is immersed in the composition, in such a way as to remove air bubbles that can form between the two electrodes of the cell. Reading the conductivity is done as soon as the value of the conductometer has stabilized. An average is taken over at least three successive measurements.

The aqueous phase comprises water. It may also comprise at least one water-soluble solvent.

The term "water-soluble solvent" denotes in the present invention a water-miscible liquid compound at ambient temperature.

The water-soluble solvents suitable for use in the compositions according to the invention may further be volatile.

Of the water-soluble solvents suitable for use in the compositions according to the invention, mention may notably be made of lower monoalcohols having 2 to 5 carbon atoms such as ethanol and isopropanol, polyols having 3 to 8 carbon atoms such as propylene glycol, 1,3-butylene glycol, caprylyl glycol, pentyleneglycol, glycerin, dipropylene glycol; C3-C4 ketones, C2-C4 aldehydes.

The aqueous phase can contain a demineralized water, or a floral water such as cornflower water and/or a mineral water such as VITTEL water, LUCAS water or LA ROCHE POSAY water and/or a spring water.

The quantity of water is at least 30% by weight with respect to the total weight of the composition. Preferably it varies from 40 to 85% by weight, and more preferably from 45 to 80% by weight with respect to the total weight of the composition.

The aqueous phase (water and optionally the water-miscible solvent) is preferably present in the composition according to the present application with a content ranging from 40% to 85% by weight, with respect to the total weight of the composition, preferably ranging from 45% to 80% by weight with respect to the total weight of the composition.

Lamellar phase Lβ formed by a surfactant system structuring the aqueous phase

The composition according to the invention comprises a lamellar phase Lβ formed by a surfactant system structuring the aqueous phase.

This lamellar phase can be:

either a lamellar phase Lβ formed by a surfactant system present in a content greater than or equal to 9% by weight

with respect to the total weight of the composition, and said surfactant system comprising at least one anionic surfactant having an HLB at 25°C greater than or equal to 8 and at least one nonionic surfactant with an HLB less than 8 at 25°C (hereinafter "ionic lamellar phase"),

or an Lβ lamellar phase formed by a surfactant system present with a content greater than or equal to 15% by weight with respect to the total weight of the composition, and said surfactant system comprising at least 5% by weight with respect to the total weight of the composition of a nonionic surfactant having an HLB at 25°C greater than or equal 8, and at least 5% by weight with respect to the total weight of a composition of a nonionic surfactant having an HLB at 25°C of less than 8 (hereinafter "nonionic lamellar phase").

The surfactant system in accordance with the invention organizes the aqueous phase in the form of a lamellar phase Lβ, or paracrystalline phase Lβ, or gel lamellar phase.

This composition is stable at an ambient temperature of 25°C. Preferably it has a viscosity ranging from 5 to 50 Pa.s, measured at an ambient temperature of 25°C using a Rhéomat RM100®.

The term gel lamellar phase or paracrystalline phase Lβ, a phase in which the molecules of surfactants and/or more generally amphiphilic compounds are organized in the form of biomolecular layers spaced by aqueous sheets. Within the biomolecular layers, the molecules are distributed according to a hexagonal structure, their hydrocarbon chains are in a crystalline state and are oriented perpendicularly to the plane of the biomolecular layers but do not have any specific orientation in relation to one another in the plane of these layers.

The paracrystalline phases Lβ are metastable phases within which the fatty chains are in the solid state and are arranged in a random manner in relation to one another, contrary to the paracrystalline micellar and lamellar fluid phases (Lα) within which the fatty chains are in the liquid state, and contrary to the crystalline phases within which the fatty chains are in the solid state and oriented in an ordered manner with respect to one another.

In order to identify the gel lamellar phase or paracrystalline phase Lβ of the surfactant system present in the composition of the invention, different techniques can be used, and in particular the technique of X-ray scattering.

*Wide angle X-ray Scattering (WAXS)*

X-ray diagrams have been recorded by an image plate detector Mar345 (Maresearch, Norderstedt, Germany), mounted on an X-ray generator with a rotating anode FR591 (Bruker, Courtaboeuf, France), used at 50 kV and at 50 mA. The monochromatic CuKα radiation ($\lambda$ = 1.541 Å) was focused with a focal spot of 350 μm to 320 mm via a double reflection on a Montel multilayer mirror with an elliptic transversal section (Incoatec, Geesthacht, Germany). The beam was defined in a vacuum by four motorized carbon-tungsten slots (JJ-Xray, Roskilde, Denmark) positioned in front of the mirror (500 μm). Four additional protective slots were placed at the focal point at a slot separation distance of 220 mm. The downstream flow of the mica output windows was 3 x $10^8$ photons/s. A beam barrier made of circular metal wire 2 mm in diameter was placed in the air at 150 mm downstream of the sample and the detector was positioned at 360 mm. The X-ray diagrams were consequently recorded for a reciprocal spacing range $q = 4\pi * \sin\theta/\lambda$ of 0.03-1.8 $Å^{-1}$, where $\theta$ is the diffraction angle. The recurring distances $d = 2\pi/q$ should be located between 200 Å and 3.5 Å. The samples were placed in glass capillaries of 1.2-1.3 mm (Glas W. Müller, Germany) and introduced in a homemade capillary fastening device, which can contain up to 20 capillaries at a controlled temperature.

*Ionic lamellar phase*

The surfactant system of the ionic lamellar phase comprises at least one anionic surfactant of HLB greater than or equal to 8 at 25°C and at least one nonionic surfactant of HLB less than 8 at 25°C.

This surfactant system structuring the aqueous phase as a lamellar phase Lβ is present with a content greater than or equal to 9% by weight with respect to the total weight of the composition, preferably 10 to 40% by weight, better from 11 to 35% by weight with respect to the total weight of the composition. In other words, the surfactant system comprises a total content in surfactant(s) greater than or equal to 10% by weight, preferably from 10% to 40% by weight, better from 11% to 35% by weight with respect to the total weight of the composition.

The surfactant system comprises at least one anionic surfactant of HLB greater than or equal to 8 at 25°C. It advantageously comprises a total content in anion surfactant(s) of HLB greater than or equal to 8 at 25°C, greater than or equal 4% by weight, in particular from 5 to 30% by weight, better from 5 to 20% by weight with respect to the total weight of the composition.

Preferably, the anionic surfactant: of HLB at 25°C greater than or equal to 8 has an HLB greater than or equal to 10.

The HLB (hydrophilic-lipophilic balance) value as per GRIFFIN is defined in J. Soc. Cosm. Chem. 1954 (volume 5), pages 249-256. Reference may be made to the document "Encyclopedia of Chemical Technology, KIRK-OTHMER", volume 22, p. 333-432, 3rd edition, 1979, WILEY, for the definition of the properties and functions of surfactants, in

particular on p. 347-377 of this reference.

Preferably, it is chosen from fatty acid salts, acyl glutamic acids and the salts thereof, and alkyl phosphates and the salts thereof, together forming a lamellar phase Lβ.

The fatty acid salts are in particular fatty monoacid salts, preferably in C12-22, such as stearic acid, palmitic acid or myristic acid preferably stearic acid. The salts are obtained typically by neutralization with a mineral or organic base.

Preferably, the acyl glutamic acids are in particular acyl glutamic acids comprising C12-22 acyl chain, and in particular among lauroyl glutamic acid, myristoyl glutamic acid, palmitoyl glutamic acid or stearoyl glutamic acid.

The alkyl phosphates are in particular C14-24 alkyl phosphates, preferably in C16-18. Preferably, the alkyl phosphate is cetyl phosphate, stearyl phosphate or cetyl phosphate. The salts are obtained typically by neutralization with a mineral or organic base. A preferred example of alkyl phosphate salt is cetyl phosphate salt.

Advantageously, the fatty acid salts or the acyl glutamic acids are chosen from: the salts obtained with alkaline metals such as sodium or potassium, preferably among sodium and potassium; and
the salts obtained with organic bases such as triethanolamine (TEA) or aminomethylpropanediol (AMPD).

Advantageously, the alkylphosphate salts are chosen from the salts obtained with alkaline metals such as sodium or potassium.

Preferably, the anionic surfactant: of HLB at 25°C greater than or equal to 8, in particular greater than or equal to 10, is stearic acid, palmitic acid and/or myristic acid neutralized with an organic base such as AMPD. Preferably, the anionic surfactant: of HLB at 25°C greater than or equal to 8, in particular greater than or equal to 10, is a mixture of stearic acid, palmitic acid and myristic acid, each acid neutralized with an organic base such as AMPD.

Preferably, according to another embodiment, the anionic surfactant having an HLB at 25°C greater than or equal to 8 is a cetyl phosphate salt, preferably potassium cetyl phosphate.

According to the invention, the ionic lamellar phase results from the association of an anionic surfactant as mentioned hereinabove with a nonionic surfactant of HLB less than 8 at 25°C. Thus, according to the invention, the surfactant system comprises at least one anionic surfactant as mentioned hereinabove, and at least one nonionic surfactant of HLB less than 8 at 25°C.

According to the invention, the nonionic surfactant is chosen from nonionic surfactants of low HLB, i.e. of HLB less than 8 at 25°C, and advantageously is chosen from:

- the fatty alcohols in $C_{14}$-$C_{22}$, preferably in $C_{16}$-$C_{18}$, such as cetyl alcohol, stearyl alcohol or cetearylic alcohol;
- esters and ethers of (poly)oxyalkylene oses;
- fatty acid esters, in particular in $C_8$-$C_{24}$, and preferably in $C_{12}$-$C_{20}$ or in $C_{16}$-$C_{22}$, and (poly)glycerol, preferably comprising one or two glycerol groups, preferably said fatty acid comprising a linear or branched, saturated or unsaturated $C_{12}$-$C_{20}$ alkyl chain, preferably linear and saturated; preferably said fatty acid ester and (poly)glycerol is chosen from glycerol stearate, glyceryl laurate, glyceryl myristate, preferably is glycerol stearate;
- the (poly)oxyalkylenated alcohols comprising a C8-C24 fatty alcohol ether and polyethylene glycol, said ether comprising from 1 to 6, better between 2 and 6, ethylene glycol units;
- and mixtures thereof.

(Poly)oxyalkylenated means from 1 to 6 oxyethylene groups (or units, better from 2 to 6 oxyethylene groups. The term "fatty acid" means preferably a fatty monoacid.

Preferably, the composition according to the invention includes a surfactant system such that:

* the anionic surfactant having an HLB greater than or equal to 8 at 25°C is stearic acid, palmitic acid and/or myristic acid neutralized with an organic base such as AMPD, or the anionic surfactant with an HLB greater than or equal to 8 at 25°C is a cetyl phosphate salt, preferably potassium cetyl phosphate; and/or
* the nonionic surfactant with an HLB less than 8 at 25°C is chosen from fatty alcohols in $C_{14}$-$C_{22}$, preferably in $C_{16}$-$C_{18}$, such as cetyl alcohol, stearyl alcohol or cetearylic alcohol.

Preferably, the composition according to the invention includes a surfactant system of the ionic lamellar phase such that:

* the concentration in anionic surfactant of HLB at 25°C greater than or equal to 8, is greater than or equal to 4% by weight, preferably between 5% and 30% by weight and even more preferably between 5% and 20% by weight with respect to the total weight of the composition, and/or
* the concentration nonionic surfactant of HLB at 25°C less than 8, is greater than or equal to 4% by weight, preferably between 5% and 30% by weight and even more preferably between 5% and 20% by weight with respect to the total weight of the composition.

*Nonionic lamellar phase*

The surfactant system of the nonionic lamellar phase comprises at least two nonionic surfactants, such that the composition comprises at least 5% by weight with respect to the total weight of the composition of a nonionic surfactant having an HLB at 25°C less than or equal to 8, and at least 5% by weight with respect to the total weight of the composition of a nonionic surfactant having an HLB at 25°C of less than 8 gelling the aqueous phase.

This surfactant system structuring the aqueous phase as a lamellar phase Lβ is present with a content greater than or equal to 15% by weight with respect to the total weight of the composition, preferably 15 to 40% by weight, better from 16 to 35% by weight with respect to the total weight of the composition. In other words, the surfactant system comprises a total content of surfactants greater than or equal to 15% by weight, preferably from 15% to 45% by weight, better from 16% to 40% by weight with respect to the total weight of the composition.

The surfactant system comprises at least one nonionic surfactant having an HLB at 25°C greater than or equal to 8, preferably greater than or equal to 10. The surfactant system also comprises at least one nonionic surfactant having an HLB at 25°C less than 8.

The HLB (hydrophilic-lipophilic balance) value as per GRIFFIN is defined in J. Soc. Cosm. Chem. 1954 (volume 5), pages 249-256. Reference may be made to the document "Encyclopedia of Chemical Technology, KIRK-OTHMER", volume 22, p. 333-432, 3rd edition, 1979, WILEY, for the definition of the properties and functions of surfactants, in particular on p. 347-377 of this reference.

The surfactants that constitute the surfactant system according to the invention can be chosen from:

- esters and ethers of polyoxyalkylenated or non-polyoxyalkylenated monosaccharides, including polyoxyalkylenated sorbitol ethers,
- polyoxyalkylenated glycerol ethers,
- polyoxyalkylenated or non-polyoxyalkylenated polyol esters. Polyoxyalkylenated polyol esters are preferred, particularly polyoxyalkylenated polyol ester, polyoxyalkylenated glycerol, polyoxyalkylenated sorbitol or polyoxyalkylenated polyethylene glycol, and
- polyoxyalkylenated or non-polyoxyalkylenated alcohols. Polyoxyalkylenated alcohols are preferred. Preferred alcohols are C8-24 fatty alcohol ethers or polyethylene glycol ethers with from 1 to 250 and preferably from 2 to 200 units of ethylene glycol. Fatty alcohols are another possible alternative, for example $C_{14-24}$ fatty alcohols and preferably $C_{14-22}$ fatty alcohols, preferably $C_{16-18}$ fatty alcohols, including a saturated or unsaturated linear or branched hydrocarbon chain, preferably, primary fatty alcohols that present a saturated linear chain such as particularly cetylic alcohol, stearylic alcohol, cetearylic alcohol, behenyl alcohol, arachidyl alcohol, lignocerylic alcohol and the mixtures thereof.

Preferably, the surfactant system comprises a nonionic surfactant with the following general formula (I):
[Chem 1]

$$(ALK_1\text{-}[C(O)]_a\text{-}[O]_b)_c\text{-}Z. \qquad (I)$$

wherein:

- ALK1 is a C7-23, preferably a C11-21 and more preferably C15-19 alkyl group,
- a and b are integers between 0 and 100, a and b are preferably equal to 0,
- c is an integer between 1 and 100, particularly between 1 and 3 and preferably equal to 1,
- Z is a group substituted or terminated by a hydroxyl group:
    - Z is preferably an oxyethylene $(CH_2CH_2O)_q$ or $(OCH_2CH_2)_q$ group in which q is an integer greater than or equal to 1. Preferably the Z group is a polyethylene glycol or is chosen from among polyglycerols,
    - Z is preferably chosen from among formulas (G) and (M):

[Chem 2]

$$HO\text{-}(ALK_2\text{-}O)_s\text{-}CH_2\text{-}CH(OALK_2)_t\text{-}OH]\text{-}CH_2\text{-}(O\text{-}ALK_2)_u\text{-}(*). \qquad (G)$$

Formula in which:

- ALK2, identical or different and represent a C1-6 alkylene group, particularly C1-4 and preferably ethylene,
- s, t and u are integers between 0 and 250, it being understood that s+t+u is not equal to 0 and is preferably comprised

between 1 and 250,

[Chem 3]

$H-(ALK_2-O)_u-(*)$.        (M)

and
[Chem 4]

$H-(O-ALK_2)_u-(*)$.        (M)

and preferably
[Chem 5]

$H-(ALK_2-O)_u-(*)$.        (M)

Formulas in which:

- SLK2, identical or different, represent a C1-6 alkylene group, particularly C1-4 and preferably ethylene.
- u represents an integer not equal to 0 and preferably between 1 and 250.

Preferably, the surfactant system comprises a nonionic surfactant with the following general formula (III):
[Chem 6]

$ALK_3-(O-CH_2-CH_2)_v-OH$.        (III)

in which

- ALK3 is a C8-24, preferably a C12-22 and more preferably a C16-18 alkyl group.
- v is an integer between 1 and 250, and preferably between 2 and 200.

Preferably, the nonionic surfactant having an HLB at 25°C less than 8 is chosen from among polyoxyalkylenated alcohols, preferably oxyethylene and/or oxypropylene alcohols with 1 to 6 ethylene glycol and/or propylene glycol units, preferably C8-24 and particularly $C_{16-\ -24}$ ethoxylated fatty alcohols, such as ethoxylated stearyl alcohol comprising 2 oxyethylene units (INCI name: steareth-2) such as Brij 72 from Uniqema; and fatty alcohols in $C_{14-24}$ and preferably in $C_{14-22}$, preferably in $C_{16-18}$, such as in particular cetyl alcohol, stearyl alcohol or cetearylic alcohol

Preferably, the nonionic surfactant having an HLB at 25°C greater than or equal to 8 is chosen from among:

- fatty acid esters (particularly C8-C24 acid, and preferably C16-C22 acid) and oxyethylene and/or oxypropylene glycerol ethers (that may include 6 to 250 oxyethylanated and/or oxypropylene groups), such as PEG-200 glyceryl monostearate sold particularly under the name Simulsol 220 TM by SEPPIC, or PEG-100 stearate sold under the name Myrj S 100 by Croda; polyethoxylated glyceryl stearate with 30 ethylene oxide groups such as the TAGAT S product sold by GOLDSCHMIDT, polyethoxylated glyceryl oleate with 30 ethylene oxide groups such as TAGAT O product sold by GOLDSCHMIDT, polyethoxylated glyceryl cocoate with 30 ethylene oxide groups such as VARIONIC LI 13 product sold by SHEREX, polyethoxylated glyceryl isostearate with 30 ethylene oxide groups such as the TAGAT L product sold by GOLDSCHMIDT; and
- oxyethylene and/or oxypropylene ethers (that may include from 6 to 250 oxyethylene and/or oxypropylene groups) of fatty alcohols (particularly C8-C24 and preferably C12-C18 alcohols) such as oxyethylene ether of stearyl alcohol with 20 oxyethylene groups (CTFA name "Steareth-20") such as BRIJ 78 marketed by UNIQUEMA, oxyethylene ether of cetearylic alcohol with 30 oxyethylene groups (CTFA name "Ceteareth-30"), and oxyethylene ether of the mix of C12-C15 fatty alcohols including 7 oxyethylene groups (CTFA name "C12-15 Pareth-7") marketed under the name NEODOL 25-7 by SHELL CHEMICALS.

According to the invention, the nonionic lamellar phase is the result of a combination of a nonionic surfactant having an HLB at 25°C greater than or equal to 8, with a nonionic surfactant having an HLB at 25°C less than 8. Thus, the surfactant system according to the invention comprises at least one nonionic surfactant having an HLB at 25°C greater than or equal to 8, with at least one a nonionic surfactant having an HLB at 25°C equal to less than 8.

According to one preferred embodiment, the surfactant system comprises a mixture of at least two polyoxyalkylenated alcohols, preferably at least two C8-24 fatty alcohol ethers or at least two polyethylene glycol ethers with 2 to 250 ethylene glycol units.

Preferably, the nonionic surfactant having an HLB of less than 8 at 25°C chosen from C16-24 ethoxylated fatty alcohols (comprising 1 to 6 ethylene glycol units), such as ethoxylated stearyl alcohol with 2 oxyethylene units (INCI name: steareth-2) such as Brij 72 from Uniqema, and the fatty alcohols in C14-C24, preferably in $C_{16}$-$C_{18}$, such as cetyl alcohol, stearyl alcohol or cetearylic alcohol.

Preferably, the nonionic surfactant having an HLB at 25°C greater than or equal to 8 is a mixture of:

- at least one C16-22 fatty acid ester and of oxyethylene glycerol ethers including 10 to 250 oxyethylenated groups, such as polyethoxylated glyceryl stearate with 30 ethylene oxide groups such as the TAGAT S product marketed by GOLDSCHMIDT, and
- at least one oxyethylene ether, including from 10 to 250 oxyethylene groups, of C12-18 fatty alcohols, such as oxyethylene ether of stearyl alcohol with 20 oxyethylene groups (CTFA name "Steareth-20") such as BRIJ 78 marketed by UNIQUEMA.

Alternatively, preferably, the nonionic surfactant having an HLB at 25°C greater than or equal to 8 is chosen from among fatty acid esters (in particular C8-C24 acid, and preferably C16-C22 acid) and oxyethylenated and/or oxypropylated glycerol ethers (that can comprise from 10 to 250 oxyethylenated and/or oxypropylated groups), such as PEG-200 glyceryl monostearate or PEG-100 stearate.

Preferably, the surfactant system comprises a mixture of:

- at least one nonionic surfactant having an HLB of less than 8 at 25°C chosen from C16-24 ethoxylated fatty alcohols (comprising 1 to 6 ethylene glycol units), such as ethoxylated stearyl alcohol with 2 oxyethylene units (INCI name: steareth-2) such as Uniqema's Brij 72; and
- at least two nonionic surfactants having an HLB at 25°C greater than or equal to 8 which are a mixture of:
- at least one C16-22 fatty acid ester and of oxyethylene glycerol ethers including 10 to 250 oxyethylenated groups, such as polyethoxylated glyceryl stearate with 30 ethylene oxide groups such as the TAGAT S product marketed by GOLDSCHMIDT, and
- at least one oxyethylene ether, including from 10 to 250 oxyethylene groups, of C12-18 fatty alcohols, such as oxyethylene ether of stearyl alcohol with 20 oxyethylene groups (CTFA name "Steareth-20") such as BRIJ 78 marketed by UNIQUEMA.

Alternatively, preferably, the surfactant system comprises a mixture of:

- at least one nonionic surfactant with an HLB less than 8 at 25°C chosen from fatty alcohols in C14-C22, preferably in C16-C18, such as cetyl alcohol, stearyl alcohol or cetearylic alcohol; and
- at least one nonionic surfactant having an HLB at 25°C greater than or equal to 8 chosen from among fatty acid esters (in particular C8-C24 acid, and preferably C16-C22 acid) and oxyethylenated and/or oxypropylated glycerol ethers (that can comprise from 10 to 250 oxyethylenated and/or oxypropylated groups), such as PEG-200 glyceryl monostearate or PEG-100 stearate.

Preferably, the composition according to the invention includes a surfactant system of the nonionic lamellar phase such that:

* the concentration in nonionic surfactant of HLB at 25°C greater than or equal to 8, is greater than or equal to 6% by weight, preferably between 7% and 30% by weight and even more preferably between 10% and 20% by weight with respect to the total weight of the composition, and/or
* the concentration nonionic surfactant of HLB at 25°C less than 8, is greater than or equal to 6% by weight, preferably between 7% and 30% by weight and even more preferably between 10% and 20% by weight with respect to the total weight of the composition.

<u>Hydrophilic gelling agent</u>

The composition according to the invention can comprise at least one hydrophilic gelling agent.

The term "hydrophilic gelling agent" means in the context of this invention a compound able to gel the aqueous phase of the compositions according to the invention. More particularly the function held by these hydrophilic gelling agents is to structure the aqueous phase of the composition. The gelling agent is advantageously soluble in the aqueous phase of the composition. The gelling agent that can be used according to the invention, can in particular be characterized

by its capacity to form in water, beyond a certain concentration C*, a gel characterized by oscillatory rheology ($\mu$ = 1 Hz) by a flow threshold $\tau c$ at least equal to 10 Pa. This concentration C* can vary greatly according to the nature of the gelling agent considered.

The hydrophilic gelling agents can be chosen from carboxyvinyl polymers such as Carbopols® (Carbomers) and Pemulen® (Coploymer Acrylates/$C_{10}$-$C_{30}$Alkylacrylate); polyacrylamides such as for example cross-linked copolymers sold under the names Sepigel 305® (INCI name: Polyacrylamide/$C_{13}$-$C_{14}$Isoparaffin/Laureth 7) or Simulgel 600® (INCI name: Acrylamide / Sodium Acryloyldimethyltaurate Copolymer / Isohexadecane / Polysorbate 80) by Seppic; 2-acrylamido 2-methylpropane sulfonic acid polymers and copolymers, optionally cross-linked and/or neutralized, such as poly(2-acrylamido 2-methylpropane sulfonic acid) marketed by Hoechst under the trade name "Hostacerin AMPS®" (INCI name: Ammonium Polyacryloyldimethyl Taurate or SIMULGEL 800® marketed by the company SEPPIC (INCI name: Sodium Polyacryolyldimethyl Taurate / Polysorbate 80 / Sorbitan Oleate); 2-acrylamido 2-methylpropane sulfonic acid and hydroxyethyl acrylate copolymers such as SIMULGEL NS® and SEPINOV EMT 10® marketed by SEPPIC; cellulose derivatives such as hydroxyethylcellulose; polysaccharides and particularly gums such as xanthan gum or hydroxypropyl guar gum.

Preferably, cellulose compounds, in particular hydroxypropyl guar gum, will be used.

The hydrophilic gelling agent or agents are preferably present in the composition in a total quantity ranging from 0.1% to 10%, preferably from 0.5% to 5% by weight with respect to the total weight of said composition.

<u>Fatty phase</u>

The composition according to the invention can comprise a fatty phase.

In this case, the fatty phase is a fatty phase dispersed in the continuous aqueous phase.

The fatty phase can be present in a content from 5% to 45% by weight, preferably from 10 to 45%, better from 10 to 40% by weight with respect to the total weight of the composition.

The term "fatty phase" means a phase comprising at least one fatty body such as oils and waxes, preferably waxes, as well as possibly all organic solvents and ingredients that are soluble or miscible in said phase when it is in the liquid state and if said fatty phase becomes solid again at ambient temperature said organic solvents and ingredients provide a macroscopically homogeneous mixture.

Preferably, the fatty phase comprises at least one wax.

*Waxes*

The composition according to the invention can comprise at least one wax. This wax makes it possible to obtain thick and loading textures.

The term "wax" refers to a lipophilic compound, which is solid at ambient temperature (25°C), deformable or not, having a reversible solid/liquid change of state and a melting point greater than or equal to 40°C that can range up to 120°C. In particular, the waxes suitable for the invention may have a melting point greater than or equal to 45°C, and particularly greater than or equal to 55°C.

The term "lipophilic compound" compound means a compound that has an acid index and a hydroxyl index less than 150 mg KOH/g.

According to the invention, the melting point is equivalent to the temperature of the most endothermic peak observed in thermal analysis (DSC) as described in the standard ISO 11357-3; 1999. The melting point of the wax may be measured using a differential scanning calorimeter (DSC), for example the calorimeter sold under the name "MDSC 2920®" by TA Instruments.

The measurement protocol is as follows:

A 5 mg sample of wax placed in a crucible is subjected to a first temperature rise from -20°C to 100°C, at a heating rate of 10°C / minute, and is then cooled from 100°C to -20°C at a cooling rate of 10°C / minute and finally subjected to a second temperature rise from -20°C to 100°C at a heating rate of 5°C / minute. During the second temperature rise, the variation in the difference in power absorbed by the empty crucible and by the crucible containing the wax sample as a function of temperature is measured. The melting point of the compound is the value of the temperature equivalent to the top of the peak of the curve representing the variation in the difference in power absorbed as a function of temperature.

The waxes may be hydrocarbon, silicone and/or fluorinated and be of plant, mineral and/or synthetic origin.

The wax or waxes are present, preferably in a content of at least 5% by weight, more preferably in a content ranging from 5 to 45% by weight with respect to the total weight of the composition, better ranging from 8 to 40% and even better from 10 to 40% by weight with respect to the total weight of the composition.

Hydrocarbon waxes such as beeswax, lanolin wax; rice wax, Carnauba wax, Candellila wax, Ouricury wax, Japan wax, Berry wax, shellac wax and sumac wax; montan wax can be in particular used as wax.

Mention may also be made of waxes obtained by means of the catalytic hydrogenation of animal or plant oils

having $C_8$-$C_{32}$ linear or branched fat chains.

Of these, particular mention may be made of hydrogenated jojoba oil, hydrogenated palm oil, hydrogenated sunflower oil, hydrogenated castor oil, hydrogenated coconut oil, hydrogenated lanolin oil, di-(trimethylol-1,1,1 propane) tetrastearate sold under the name "HEST 2T-4S®" by HETERENE, di-(trimethylol-1,1,1 propane) tetrabehenate sold under the name HEST 2T-4B® by HETERENE.

The wax used can also be obtained by hydrogenating esterified olive oil with stearyl alcohol sold under the name "PHYTOWAX Olive 18 L 57®" or waxes obtained by hydrogenating esterified castor oil with cetyl alcohol sold under the name "PHYTOWAX ricin 16L64® and 22L73®", by SOPHIM. Such waxes are described in the application FR-A-2792190.

A C20-C40 alkyl (hydroxystearyloxy)stearate (the alkyl group comprising 20 to 40 carbon atoms), alone or in a mixture, may also be used, in particular a C20-C40 alkyl 12-(12'-hydroxystearyloxy) stearate having the formula hereinbelow:

[Chem 7]

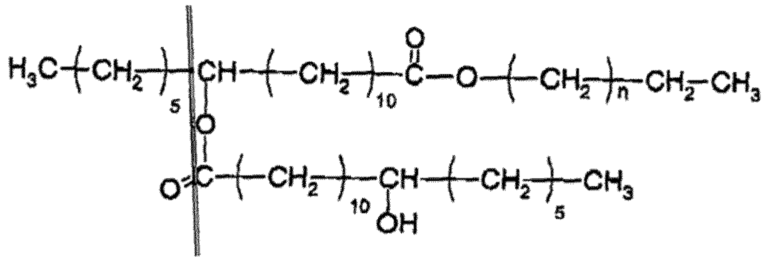

wherein n is an integer ranging from 18 to 38, or a mixture of compounds having the formula hereinabove. Such a sticky wax is particularly sold under the names "KESTER WAX K 82 P®" and "KESTER WAX K 80 P®" by KOSTER KEUNEN.

Mention can be made of microcrystalline waxes, paraffins and ozokerite; polyethylene waxes, waxes obtained by means of Fisher-Tropsch synthesis and waxy copolymers and the esters thereof; silicone waxes and fluorinated waxes.

Mention can be made of mono-esters of linear fatty acid having the following formula:

[Chem 8] $R_3$-O-$R_4$

in which $R_3$ and $R_4$ are linear and saturated and have, independently of one another, a number of carbon atoms greater than or equal to 20, with $R_3$ representing an acyl radical, and $R_4$ representing an alkyl radical.

In particular, the mono-ester of fatty acids according to the invention is chosen from arachidyl arachidate and behenyl behenate and more particularly behenyl behenate.

According to a preferred embodiment, a wax will be used chosen from beeswax, Carnauba wax, cetyl alcohol, paraffins, behenyl behenate and mixtures thereof.

<u>Non-volatile oils</u>

The composition according to the invention can comprise at least one non-volatile oil.

The term "oil" refers to a non-aqueous compound, which is not water-miscible, and liquid at ambient temperature (25°C) and atmospheric pressure (760 mm Hg).

The term "non-volatile oil" denotes an oil remaining on the keratin fiber at ambient temperature and atmospheric pressure for at least several hours and particularly having a vapor pressure less than $10^{-3}$ mm Hg (0.13 Pa).

These oils may be hydrocarbon oils, silicone oils, or mixtures thereof.

A "hydrocarbon oil" is an oil containing principally hydrogen and carbon atoms and possible oxygen, nitrogen, sulfur and phosphorus atoms.

For the purposes of this invention, the term "silicone oil" denotes an oil comprising at least one silicon atom, and in particular at least one Si-O group.

As a non-volatile hydrocarbon oil, mention may be made of:

- hydrocarbon oils of plant origin such as triesters of fatty acids and glycerol for which the fatty acids can have chain lengths ranging from $C_4$ to $C_{24}$, with the latter able to be linear or branched, saturated or unsaturated; these oils are in particular wheat germ, sunflower, grape seed, sesame, corn, apricot, castor, shea, avocado, olive, soybean oils, sweet almond, palm, rapeseed, cotton, hazelnut, macadamia, jojoba, alfalfa, poppy seed, pumpkin, sesame, squash, rapeseed, blackcurrant, evening primrose, millet, barley, quinoa, rye, safflower, candlenut, passiflora, musk rose oil; or caprylic/capric acid triglycerides such as those sold by Stearineries Dubois or those sold under the trade names

Miglyol 810®, 812® and 818® by Dynamit Nobel;

- linear or branched hydrocarbons of mineral or synthetic origin, such as liquid paraffins and derivatives thereof, polydecenes, polybutenes, hydrogenated polyisobutene such as Parleam, squalane;
- synthetic ethers having from 10 to 40 carbon atoms;
- synthetic esters such as oils having the formula $R_1COOR_2$ in which $R_1$ represents the residue of a linear or branched fatty acid including 1 to 40 carbon atoms and $R_2$ represents a hydrocarbon chain, particularly branched containing 1 to 40 carbon atoms on the condition that $R_1 + R_2$ is $\geq 10$, such as for example Purcellin oil (cetostearyl octanoate), isopropyl myristate, isopropyl palmitate, $C_{12}$ to $C_{15}$ alcohol benzoates, hexyl laurate, diisopropyl adipate, isononyl isononanoate, 2-ethylhexyl palmitate, isostearyl isostearate, heptanoates, octanoates decanoates or ricinoleates of alcohols or polyalcohols such as propylene glycol dioctanoate; hydroxylated esters, such as isostearyl lactate, diisostearyl malate; polyol esters and pentaerythritol esters;
- fatty alcohols that are liquid at ambient temperature, with a branched and/or unsaturated carbon chain having 12 to 26 carbon atoms, such as octyldodecanol, isostearyl alcohol, oleic alcohol, 2-hexyldecanol, 2-butyloctanol, 2-undecylpentadecanol;
- higher fatty acids, such as oleic acid, linoleic acid, linolenic acid;

and mixtures thereof.

The non-volatile silicone oils that can be used in the composition in accordance with the invention can be non-volatile polydimethylsiloxanes (PDMS), polydimethylsiloxanes including alkyl or alkoxy groups which are pendant or at the end of the silicone chain, groups each having 2 to 24 carbon atoms; phenylated silicones, such as phenyl trimethicones, phenyl dimethicones, phenyltrimethylsiloxydiphenylsiloxanes, diphenyl dimethicones, diphenylmethyldiphenyl-trisiloxanes or (2-phenylethyl)trimethylsiloxysilicates.

According to a preferred form of the invention, the non-volatile oil is present in the composition in a total concentration less than 10% by weight, preferably less than 5% by weight with respect to the total weight of the composition. Preferably, the composition is free from non-volatile oil.

Dye

According to a particular form of the invention, the composition according to the invention comprises in addition at least one dye, in particular chosen from powder dyes, water-soluble dyes, and the mixtures thereof.

The powder dyes can be chosen from pigments and nacres.

The pigments may be white or colored, inorganic and/or organic, coated or uncoated. Of the inorganic pigments, mention may be made of titanium dioxide, optionally surface-treated, zirconium, zinc or cerium oxides, along with iron or chromium oxides, manganese violet, ultramarine blue, chromium hydrate and iron blue. Of the organic pigments, mention may be made of carbon black, D & C type pigments, and lacquers based on cochineal carmine, barium, strontium, calcium, aluminum.

The nacres may be chosen from white pearlescent pigments such as mica coated with titanium, or bismuth oxychloride, colored pearlescent pigments such as titanium mica with iron oxides, titanium mica with iron blue and chromium oxide in particular, titanium mica with an organic pigment of the aforementioned type and pearlescent pigments based on bismuth oxychloride.

Preferably, the composition according to the invention comprises a powder dye, preferably of the pigment type, in particular metal oxides, in particular titanium dioxides, iron oxides and the mixtures thereof; and more particularly iron oxides.

Preferably, said dye such as the pigment or pigments are present in the composition in a content ranging from 2 to 25% by weight, preferably from 3% to 20% by weight, more particularly from 4 to 15% by weight with respect to the total weight of the composition.

As water-soluble dyes suitable for the invention, mention can in particular be made of synthetic or natural water-soluble dyes such as for example FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beet), carmine, copper chlorophyllin, methylene blue, lanthocyanins (enocianin, black carrot, hibiscus, elder flower), caramel, la riboflavin. The water-soluble or dyes are, for example, beet juice and caramel. The water-soluble or liposoluble dyes are preferably present in the composition in contents less than 4% by weight, even 2% by weight, more preferably ranging from 0.01 to 2% by weight, and even better from 0.02 to 1.5% by weight with respect to the total weight of the composition.

Additives

As cosmetic additives that can be used in the compositions according to the invention, particular mention can be made of antioxidants; preservatives; scents; organic or mineral fillers; sequestrants; chelating agents; solar filters;

vitamins such as vitamin B3 or niacinamide; vitamin B8 also called H: D-Biotine, vitamin E and the derivatives thereof such as tocopherol acetate; and the mixtures thereof.

These additives may be present in the composition at a content ranging from 0.01% to 15% of the total weight of the composition.

The composition according to the invention preferably comprises a content less than 10% by weight in active substance with respect to the total weight of the composition, of film-forming polymer. Preferably, the composition comprises a content less than 5% by weight in active substance with respect to the total weight of the composition, of film-forming polymer. Preferably it is substantially free of film-forming polymer. "Substantially free" means that the composition comprises less than 1%, preferably less than 0.5% by weight, preferably less than 0.1% of active substance with respect to the total composition weight of the composition, of film-forming polymer, preferably it is entirely free of film-forming polymer.

In the present application, the term "film-forming polymer" means a polymer that can form by itself or in the presence of an auxiliary film-forming agent, a continuous deposit on a support, at a temperature of 20°C.

This film-forming polymer can be in the form of solid particles, or used as is in suspension in the aqueous phase of the composition, or in the form of particles in aqueous dispersion (latex or pseudo-latex).

Obviously, those skilled in the art will make sure to choose these optional additional compounds, and/or the quantity thereof, such that the advantageous properties of the active constituents of the composition according to the invention are not, or are substantially not, altered by the envisaged addition.

According to a particular form of the invention, the composition according to the invention is in the form of a product for eyelashes such as a mascara.

Third embodiment of a kit

The invention further relates to a third embodiment of a cosmetic application kit comprising at least:

- the shaping tool 10A, 10B as described hereinabove;
- a first composition comprising at least 30% by weight of water with respect to the total weight of the composition, forming an aqueous phase, at least 1% by weight with respect to the total weight of the cysteine composition and/or one of the salts thereof, a lamellar phase Lβ formed by a surfactant system structuring the aqueous phase, and said composition having a pH greater than 8.0; the first composition having for example the characteristics of the one defined hereinabove for the second embodiment of the kit;
- an applicator of the first composition, the applicator of the first composition being separate from the shaping tool 10A, 10B and being for example as defined hereinabove for the second embodiment of the kit;
- a second cosmetic mascara composition, for example as defined hereinabove for the first embodiment of the kit;
- an applicator of the second cosmetic mascara composition, the applicator of the second cosmetic composition being separate from the shaping tool 10A, 10B and separate from the applicator of the first composition, and being for example as defined hereinabove for the first embodiment of the kit.

Shaping method

A method for shaping keratin fibers using a shaping tool 10A, 10B as described hereinabove shall now be described.

The method comprises providing a shaping tool 10A, 10B described hereinabove. The tool is for example provided in one of the three embodiments of the kit described hereinabove.

The method comprises for example a preliminary step of applying a composition on keratin fibers by a specific applicator and separate from the shaping tool 10A, 10B.

Preferably, the application is that of the composition comprising at least 30% by weight of water with respect to the total weight of the composition, forming an aqueous phase, at least 1% by weight with respect to the total weight of the cysteine composition and/or of one of the salts thereof, a lamellar phase Lβ formed by a surfactant system structuring the aqueous phase, and said composition having a pH greater than 8.0.

This composition has for example the characteristics of that of the second embodiment of the corresponding kit.

A contact time between the fiber and the composition for several minutes can be provided, for example 10 to 30 minutes, or several hours such as for example up to 8 hours.

The method also comprises curling keratin fibers by application of the shaping tool 10A, 10B on the fibers.

During this step, the fibers are received in the grooves 22 and curled by the force exerted on them by the shaping member 14.

The shaping tool 10A, 10B makes it possible, in combination with said composition, to curl the fibers in a persistent manner.

Preferably, in order to increase the curling of the keratin fibers and the persistence of the effect in particular at makeup removal, the makeup method of the invention is repeated several times by applying the composition, preferably 3 times in succession or at various moments separated in time.

As an advantageous supplement, the method also comprises the application of a cosmetic mascara composition on the fibers, for example having the characteristics of that of the first embodiment of the kit, by an applicator separate from the shaping tool 10A, 10B, for example having the characteristics of the first embodiment of the corresponding kit.

Thanks to the combination of the shaping tool 10A, 10B and of the composition comprising the cysteine and/or one of the salts thereof, it is then possible to provide a curling of the keratin fibers that is durable and persistent, in addition to the advantages of the tool itself, namely the ease of use and the absence of damage for the fibers.

In the above, the expressions "comprised between ... and ..." and "ranging from ... to ..." are to be understood to be inclusive of the limits, unless specified otherwise.

The invention is illustrated in more detail by the examples and figures shown hereinafter. Unless mentioned otherwise, the quantities indicated are expressed as a percentage by mass (% w/w).

**Example 1: Kit according to the invention for the makeup of the eyelashes: reducing composition with 5% L-cysteine in an ionic lamellar** phase

The reducing composition was carried out with 5% L-cysteine according to the following invention:

[Table 1]

| **Ingredients (INCI name)** | **Reducing composition according to the invention (% w/w) (Composition 1)** |
|---|---|
| Potassium cetyl phosphate (Amphisol K from DSM) | 12 |
| Cetyl alcohol (Lanette 16 from BASF) | 12 |
| Aminomethylpropanediol (AMPD ULTRA from Angus) | 3.5 |
| L-CYSTEINE (AJINOMOTO) | 5 |
| Water | qs 100 |

Composition preparation protocol

In a 500mL double-shell skillet with oil circulation to control the temperature, the different ingredients are added then the water heated beforehand in an electric kettle to 95°C. The skillet is heated to about 90°C.

Once melted, they are homogenized by stirring with the Moritz, with a rotor stator type stirring: it is composed of a fixed part inside which a second mobile part rotates at variable speed, this device is used to make the emulsions because it can generate a very high shear. The sample is then cooled under low stirring to return to ambient temperature.

**Example 2: Kit outside the invention for the makeup of the eyelashes: composition without L-cysteine in an ionic lamellar phase**

The following composition is carried out without L-cysteine (outside the invention, comparative):

[Table 2]

| **Ingredients (INCI name)** | **Composition outside the invention (% w/) (Composition 2)** |
|---|---|
| Potassium cetyl phosphate (Amphisol K from DSM) | 12 |
| Cetyl alcohol (Lanette 16 from BASF) | 12 |
| Water | qs 100 |

The composition was prepared in the same conditions as those of example 1.

**Example 3: Kit outside the invention for the makeup of the eyelashes: composition without L-cysteine in a nonionic lamellar phase**

The following composition is carried out without L-cysteine (outside the invention, comparative):

[Table 3]

| Ingredients (INCI name) | Composition outside the invention(% w/w) (Composition 3) |
|---|---|
| PEG100 stearate (Myrj S 100-PA-(SG) from Croda) | 15 |
| Cetyl alcohol (Lanette 16 from BASF) | 15 |
| Hydroxyethylcellulose (Cellosize HEC QP-4400H Europe PCG from Amerchol) | 1 |
| Preservative | qs |
| Water | qs 100 |

Composition preparation protocol

The composition was prepared in the same conditions as those of example 1.

**Example 4: Kit according to the invention for the makeup of the eyelashes: reducing composition with 5% L-cysteine in a nonionic lamellar phase**

The reducing composition was carried out with 5% L-cysteine according to the following invention:

[Table 4]

| Ingredients (INCI name) | Reducing composition according to the invention (Composition 4) |
|---|---|
| Composition 3 of example 3 | 91.5 |
| Aminomethylpropanediol (AMPD ULTRA from Angus) | 3.5 |
| L-CYSTEINE (AJINOMOTO) | 5 |

The composition was prepared by maxing at ambient temperature aminopropanediol and L-cysteine with the composition 3 from example 3.

**Example 5: Comparative tests of the measurement of the curling and of the persistence**

Compositions 1 to 4 are packaged in the Pack HYPNOSE® from LANCÔME®. The products were applied on test pieces of false eyelashes. The four kits 1 to 4 for the makeup of the eyelashes of examples 1 to 4 that were implemented were compared.

Each composition was applied on a test piece of false eyelashes, using the applicator (brush) used in the Hypnôse product from Lancôme: the brush is impregnated with composition, then 30 passes of the brush on the test piece are carried out.

Then, the shaping tool according to the invention is used, to curl the false eyelashes for 20 minutes. The shaping tool according to the invention used is such that each rib has a shape with a triangular point, and it has the following dimensions:

$$P = 2mm$$

$$D = 2.5mm$$

$$\text{lateral radius of curvature } R = 80mm$$

main radius of curvature r = flat case

L = 35mm

W = 8mm

rod diameter 4mm

Following this step, the test pieces with makeup were left for at least 6 hours, then the makeup was removed from the false eyelashes with a makeup remover of the micellar solution type (for example from GARNIER).

The effect of the curling of the eyelashes and the persistence of the effect after makeup removal was then observed with the naked eye for each makeup kit 1 to 4. The results obtained are indicated the following table 5. The curling is quantified by the difference in angle indicated in the description (it is indicated hereinbelow in parentheses).

[Table 5]

| Composition tested | Observations |
|---|---|
| Composition 1 of example 1 (invention) | Gain in curling after makeup removal (29°) |
| Composition 2 of example 2 | No curling after makeup removal |
| Composition 3 of example 3 | No curling after makeup removal |
| Composition 4 of example 4 (invention) | Gain in curling after makeup removal (39°) |

It was observed that kits 1 and 4 according to the invention comprising an aqueous composition having a pH > 8.0 comprising at least 1% L-cysteine (i.e. 5%) and an ionic or nonionic lamellar phase, resulted in a significant durable and persistent curling of the eyelashes at makeup removal, contrary to kits 2 and 3 outside the invention comprising an aqueous composition having a pH > 8.0, and an ionic or nonionic lamellar phase.

The same experiment was conducted with the same compositions 1 to 4 and with a shaping tool similar to the one according to the invention, but without any tooth:

A persistent curling was observed with the compositions comprising cysteine (i.e. compositions 1 and 4), but the fringe of false eyelashes is inhomogeneous because the eyelashes are gathered together in the form of bundles.

Compositions 2 and 3 outside the invention do not lead to any persistent curling; after makeup removal, the eyelashes return to their initial position.

## Claims

1. Shaping tool (10A, 10B) for keratin fibers adapted for being used after prior application on the keratin fibers of a cosmetic composition by another applicator separate from the shaping tool (10A, 10B), the shaping tool (10A, 10B) comprising a gripping member (12) extending along a longitudinal axis (X), and a shaping member (14) comprising a base body (18) and ribs (20),

    the base body (18) being integral with the gripping member (12), and having two lateral edges (24), and extending from the gripping member (12), to a free distal edge (26) opposite the gripping member (12), the free distal edge (26) joining the two lateral edges (24) to one another,

    each rib (20) protruding from the base body (18) in a respective elevation direction (Z), the ribs (20) also extending parallel to one another and to a transverse axis (Y) and defining grooves (22) between them, each rib (20) having a constant height along the transverse axis (Y) between the two lateral edges (24) of the base body (18), the free distal edge (26) of the base body (18) extending parallel to the transverse axis (Y).

2. Tool (10A, 10B) according to claim 1, wherein the free distal edge (26) of the base body (18) stops at the rib (30A) that is farthest from the gripping member (12).

3. Tool (10A, 10B) according to any one of claims 1 or 2, wherein each lateral edge has a respective lateral radius of curvature (R) greater than or equal to 2.0 cm, in projection in a plane parallel to the longitudinal axis (A) and to the transverse axis (Y), the lowest lateral radius of curvature R being preferably comprised between 5.0 cm and 9.0 cm.

4. Tool (10A, 10B) according to any one of claims 1 to 3, wherein a separation (W) between the two lateral edges (24) of the base body (18) is constant along the base body (18), the separation being preferably comprised between 3.0 mm and 10.0 mm, advantageously between 7.0 mm and 9.0 mm.

5. Tool (10A, 10B) according to any one of claims 1 to 4, wherein each groove (22) has a bottom (32) formed by the base body (18) and all the bottoms (32) of the grooves (22) follow a shape that has a main radius of curvature (r) greater than or equal to 2.0 cm, in projection in a plane parallel to the longitudinal axis (A) and perpendicular to the transverse axis (Y).

6. Tool (10A, 10B) according to claim 5, wherein the main radius of curvature (r) is comprised between 1 cm and 10 cm in such a way that all the bottoms (32) of the grooves (22) follow a curved shape, or the main radius of curvature (r) is such that all the bottoms (32) of the grooves (22) follow a planar shape.

7. Tool (10A, 10B) according to claim 6, wherein the shaping member (14 has at least one plane of symmetry perpendicular to the longitudinal axis (A).

8. Tool (10A, 10B) according to any one of claims 1 to 7, wherein the height (D) of each rib (20) taken along the elevation direction (Z) is comprised between 1.0 mm and 4.0 mm, preferably comprised between 2.0 mm to 3.0 mm.

9. Tool (10A, 10B) according to any one of claims 1 to 8, wherein each rib (20) extends from the base body (18) along a respective elevation direction (Z) up to a top (36), the separation (P) between the tops (36) of two adjacent ribs (20) being constant and advantageously comprised between 0.3 mm and 5.0 mm, preferably comprised between 1.0 mm and 2.0 mm.

10. Tool (10A, 10B) according to any one of claims 1 to 9, wherein each rib (20) extends from the base body (18) along a respective elevation direction (Z) up to a point (36), the distance (L) between the tops (36) of the two ribs (30A, 30B) the farthest from one another being comprised between 1.0 cm and 5.0 cm, preferably comprised between 2.0 cm and 4.0 cm, advantageously comprised between 3.0 cm and 4.0 cm.

11. Tool (10A, 10B) according to any one of claims 1 to 10, wherein each rib (20) has a shape with a triangular point, in a slot, or curved, in a cross-section perpendicular to the transverse axis (Y).

12. Cosmetic application kit comprising:

- a shaping tool (10A, 10B) according to any one of claims 1 to 11;
- a cosmetic mascara composition; and
- a cosmetic composition applicator separate from the shaping tool (10A, 10B).

13. Cosmetic application kit comprising:

- a shaping tool (10A, 10B) according to any one of claims 1 to 11;
- a composition comprising at least 30% by weight of water with respect to the total weight of the composition, forming an aqueous phase, at least 1% by weight with respect to the total weight of the cysteine composition and/or one of the salts thereof, a lamellar phase $L\beta$ formed by a surfactant system structuring the aqueous phase, and said composition having a pH greater than 8.0; and
- a composition applicator separate from the shaping tool (10A, 10B).

14. Cosmetic application kit comprising:

- a shaping tool (10A, 10B) according to any one of claims 1 to 11;
- a first composition comprising at least 30% by weight of water with respect to the total weight of the composition, forming an aqueous phase, at least 1% by weight with respect to the total weight of the cysteine composition and/or one of the salts thereof, a lamellar phase $L\beta$ formed by a surfactant system structuring the aqueous phase, and said composition having a pH greater than 8.0;
- an applicator of the first composition separate from the shaping tool (10A, 10B);
- a second cosmetic mascara composition; and
- an applicator of the second cosmetic composition separate from the shaping tool (10A, 10B) and separate from the applicator of the first composition.

**15.** Method for shaping keratin fibers comprising:

- providing a shaping tool (10A, 10B) according to any one of claims 1 to 11;
- a preliminary step of applying a composition on the keratin fibers by a specific applicator, the specific applicator being separate from the shaping tool (10A, 10B);
- curling keratin fibers by application of the shaping tool (10A, 10B) on the fibers.

**16.** Makeup method for keratin fibers, in particular the eyelashes, comprising a first step of applying on said keratin fibers a composition comprising:

at least 30% by weight of water with respect to the total weight of the composition, forming an aqueous phase, at least 1% by weight with respect to the total weight of the composition of cysteine and/or one of the salts thereof, a lamellar phase Lβ formed by a surfactant system structuring the aqueous phase, and said composition having a pH greater than 8.0,

then a second step of shaping said fibers by application on the latter of a shaping tool (10A, 10B), said tool comprising a gripping member (12) extending along a longitudinal axis (X), and a shaping member (14) comprising a base body (18) and ribs (20),
the base body (18) being integral with the gripping member (12), and having two lateral edges (24), and extending from the gripping member (12) to a free distal edge (26) opposite the gripping member (12), the free distal edge (26) joining the two lateral edges (24) to one another,
each rib (20) protruding from the base body (18) in a respective elevation direction (Z), the ribs (20) also extending parallel to one another and to a transverse axis (Y) and defining grooves (22) between them.

## Patentansprüche

**1.** Formgebungswerkzeug (10A, 10B) für Keratinfasern, ausgebildet zur Verwendung nach vorherigem Auftragen einer kosmetischen Zusammensetzung auf die Keratinfasern mittels eines anderen, vom Formgebungswerkzeug (10A, 10B) getrennten Applikators, wobei das Formgebungswerkzeug (10A, 10B) ein Greifelement (12), das sich entlang einer Längsachse (X) erstreckt, und ein Formgebungselement (14), das einen Grundkörper (18) und Rippen (20) umfasst, aufweist,

wobei der Grundkörper (18) integral mit dem Greifelement (12) ausgebildet ist und zwei seitliche Ränder (24) aufweist und sich von dem Greifelement (12) bis zu einem freien distalen Rand (26) erstreckt, der dem Greifelement (12) gegenüberliegt, wobei der freie distale Rand (26) die beiden seitlichen Ränder (24) miteinander verbindet,
wobei jede Rippe (20) in einer jeweiligen Erhebungsrichtung (Z) von dem Grundkörper (18) vorsteht, wobei sich die Rippen (20) ferner parallel zueinander und zu einer Querachse (Y) erstrecken und zwischen sich Nuten (22) definieren, wobei jede Rippe (20) entlang der Querachse (Y) zwischen den beiden seitlichen Rändern (24) des Grundkörpers (18) eine konstante Höhe aufweist, wobei sich der freie distale Rand (26) des Grundkörpers (18) parallel zur Querachse (Y) erstreckt.

**2.** Werkzeug (10A, 10B) nach Anspruch 1, wobei der freie distale Rand (26) des Grundkörpers (18) an der Rippe (30A) endet, die am weitesten von dem Greifelement (12) entfernt ist.

**3.** Werkzeug (10A, 10B) nach einem der Ansprüche 1 oder 2, wobei jeder seitliche Rand einen jeweiligen seitlichen Krümmungsradius (R) von größer oder gleich 2,0 cm aufweist, in Projektion in eine Ebene parallel zur Längsachse (A) und zur Querachse (Y), wobei der kleinste seitliche Krümmungsradius R vorzugsweise zwischen 5,0 cm und 9,0 cm umfasst.

**4.** Werkzeug (10A, 10B) nach einem der Ansprüche 1 bis 3, wobei ein Abstand (W) zwischen den beiden seitlichen Rändern (24) des Grundkörpers (18) entlang des Grundkörpers (18) konstant ist, wobei der Abstand vorzugsweise zwischen 3,0 mm und 10,0 mm, vorteilhaft zwischen 7,0 mm und 9,0 mm umfasst.

**5.** Werkzeug (10A, 10B) nach einem der Ansprüche 1 bis 4, wobei jede Nut (22) einen durch den Grundkörper (18) gebildeten Boden (32) aufweist und alle Böden (32) der Nuten (22) einer Form folgen, die einen Hauptkrümmungsradius (r) von größer oder gleich 2,0 cm aufweist, in Projektion in eine Ebene parallel zur Längsachse (A) und

senkrecht zur Querachse (Y).

6. Werkzeug (10A, 10B) nach Anspruch 5, wobei der Hauptkrümmungsradius (r) zwischen 1 cm und 10 cm umfasst, so dass alle Böden (32) der Nuten (22) einer gekrümmten Form folgen, oder wobei der Hauptkrümmungsradius (r) derart ist, dass alle Böden (32) der Nuten (22) einer ebenen Form folgen.

7. Werkzeug (10A, 10B) nach Anspruch 6, wobei das Formgebungselement (14) mindestens eine senkrecht zur Längsachse (A) verlaufende Symmetrieebene aufweist.

8. Werkzeug (10A, 10B) nach einem der Ansprüche 1 bis 7, wobei die entlang der Erhebungsrichtung (Z) gemessene Höhe (D) jeder Rippe (20) zwischen 1,0 mm und 4,0 mm umfasst, vorzugsweise zwischen 2,0 mm und 3,0 mm umfasst.

9. Werkzeug (10A, 10B) nach einem der Ansprüche 1 bis 8, wobei sich jede Rippe (20) von dem Grundkörper (18) entlang einer jeweiligen Erhebungsrichtung (Z) bis zu einer Spitze (36) erstreckt, wobei der Abstand (P) zwischen den Spitzen (36) zweier benachbarter Rippen (20) konstant ist und vorteilhaft zwischen 0,3 mm und 5,0 mm, vorzugsweise zwischen 1,0 mm und 2,0 mm umfasst.

10. Werkzeug (10A, 10B) nach einem der Ansprüche 1 bis 9, wobei sich jede Rippe (20) von dem Grundkörper (18) entlang einer jeweiligen Erhebungsrichtung (Z) bis zu einer Spitze (36) erstreckt, wobei der Abstand (L) zwischen den Spitzen (36) der beiden am weitesten voneinander entfernten Rippen (30A, 30B) zwischen 1,0 cm und 5,0 cm umfasst, vorzugsweise zwischen 2,0 cm und 4,0 cm, vorteilhaft zwischen 3,0 cm und 4,0 cm umfasst.

11. Werkzeug (10A, 10B) nach einem der Ansprüche 1 bis 10, wobei jede Rippe (20) in einem Querschnitt senkrecht zur Querachse (Y) eine Form mit dreieckiger Spitze, eine schlitzförmige Form oder eine gekrümmte Form aufweist.

12. Kosmetisches Applikationskit, umfassend:

- ein Formgebungswerkzeug (10A, 10B) nach einem der Ansprüche 1 bis 11;
- eine kosmetische Mascarazusammensetzung; und
- einen von dem Formgebungswerkzeug (10A, 10B) getrennten Applikator für die kosmetische Zusammensetzung.

13. Kosmetisches Applikationskit, umfassend:

- ein Formgebungswerkzeug (10A, 10B) nach einem der Ansprüche 1 bis 11;
- eine Zusammensetzung, die mindestens 30 Gew.-% Wasser bezogen auf das Gesamtgewicht der Zusammensetzung umfasst und eine wässrige Phase bildet, mindestens 1 Gew.-% bezogen auf das Gesamtgewicht der Zusammensetzung an Cystein und/oder einem seiner Salze, eine lamellare Phase Lß, die durch ein die wässrige Phase strukturierendes Tensidsystem gebildet wird, wobei die genannte Zusammensetzung einen pH-Wert von größer als 8,0 aufweist; und
- einen von dem Formgebungswerkzeug (10A, 10B) getrennten Applikator für die Zusammensetzung.

14. Kosmetisches Applikationskit, umfassend:

- ein Formgebungswerkzeug (10A, 10B) nach einem der Ansprüche 1 bis 11;
- eine erste Zusammensetzung, die mindestens 30 Gew.-% Wasser bezogen auf das Gesamtgewicht der Zusammensetzung umfasst und eine wässrige Phase bildet, mindestens 1 Gew.-% bezogen auf das Gesamtgewicht der Zusammensetzung an Cystein und/oder einem seiner Salze, eine lamellare Phase Lß, die durch ein die wässrige Phase strukturierendes Tensidsystem gebildet wird, wobei die genannte Zusammensetzung einen pH-Wert von größer als 8,0 aufweist;
- einen von dem Formgebungswerkzeug (10A, 10B) getrennten Applikator der ersten Zusammensetzung;
- eine zweite kosmetische Mascarazusammensetzung; und
- einen Applikator der zweiten kosmetischen Zusammensetzung, der von dem Formgebungswerkzeug (10A, 10B) und von dem Applikator der ersten Zusammensetzung getrennt ist.

15. Verfahren zum Formen von Keratinfasern, umfassend:

- Bereitstellen eines Formgebungswerkzeugs (10A, 10B) nach einem der Ansprüche 1 bis 11;
- einen vorbereitenden Schritt des Auftragens einer Zusammensetzung auf die Keratinfasern mittels eines spezifischen Applikators, wobei der spezifische Applikator von dem Formgebungswerkzeug (10A, 10B) getrennt ist;
- Kräuseln von Keratinfasern durch Aufbringen des Formgebungswerkzeugs (10A, 10B) auf die Fasern.

**16.** Make-up-Verfahren für Keratinfasern, insbesondere die Wimpern, umfassend einen ersten Schritt des Auftragens einer Zusammensetzung auf die genannten Keratinfasern, die umfasst:

mindestens 30 Gew.-% Wasser bezogen auf das Gesamtgewicht der Zusammensetzung, eine wässrige Phase bildend,
mindestens 1 Gew.-% bezogen auf das Gesamtgewicht der Zusammensetzung an Cystein und/oder einem seiner Salze,
eine lamellare Phase Lß, die durch ein die wässrige Phase strukturierendes Tensidsystem gebildet wird, wobei die genannte Zusammensetzung einen pH-Wert von größer als 8,0 aufweist,
und dann einen zweiten Schritt des Formens der genannten Fasern durch Aufbringen eines Formgebungswerkzeugs (10A, 10B) auf diese, wobei das genannte Werkzeug ein Greifelement (12), das sich entlang einer Längsachse (X) erstreckt, und ein Formgebungselement (14), das einen Grundkörper (18) und Rippen (20) umfasst, aufweist,
wobei der Grundkörper (18) integral mit dem Greifelement (12) ausgebildet ist und zwei seitliche Ränder (24) aufweist und sich von dem Greifelement (12) bis zu einem freien distalen Rand (26) erstreckt, der dem Greifelement (12) gegenüberliegt, wobei der freie distale Rand (26) die beiden seitlichen Ränder (24) miteinander verbindet,
wobei jede Rippe (20) in einer jeweiligen Erhebungsrichtung (Z) von dem Grundkörper (18) vorsteht, wobei sich die Rippen (20) ferner parallel zueinander und zu einer Querachse (Y) erstrecken und zwischen sich Nuten (22) definieren.

## Revendications

**1.** Outil de mise en forme (10A, 10B) de fibres kératiniques adapté pour être utilisé après application préalable sur les fibres kératiniques d'une composition cosmétique par un autre applicateur distinct de l'outil de mise en forme (10A, 10B), l'outil de mise en forme (10A, 10B) comprenant un organe de préhension (12) s'étendant suivant un axe longitudinal (X), et un organe de mise en forme (14) comprenant un corps de base (18) et des nervures (20),

le corps de base (18) étant solidaire de l'organe de préhension (12), et présentant deux bords latéraux (24), et s'étendant depuis l'organe de préhension (12) jusqu'à un bord distal libre (26) opposé à l'organe de préhension (12), le bord distal libre (26) joignant les deux bords latéraux (24) l'un à l'autre,
chaque nervure (20) s'étendant en saillie à partir du corps de base (18) suivant une direction d'élévation (Z) respective, les nervures (20) s'étendant aussi parallèlement les unes aux autres et à un axe transversal (Y) et définissant entre elles des sillons (22), chaque nervure (20) présentant une hauteur constante le long de l'axe transversal (Y) entre les deux bords latéraux (24) du corps de base (18), le bord distal libre (26) du corps de base (18) s'étendant parallèlement à l'axe transversal (Y).

**2.** Outil (10A, 10B) selon la revendication 1, dans lequel le bord distal libre (26) du corps de base (18) s'arrête à la nervure (30A) la plus éloignée de l'organe de préhension (12).

**3.** Outil (10A, 10B) selon l'une quelconque des revendications 1 ou 2, dans lequel chaque bord latéral présente un rayon de courbure latérale (R) respectif supérieur ou égal à 2,0 cm, en projection dans un plan parallèle à l'axe longitudinal (A) et à l'axe transversal (Y), le rayon de courbure latérale R le plus faible étant préférentiellement compris entre 5,0 cm et 9,0 cm.

**4.** Outil (10A, 10B) selon l'une quelconque des revendications 1 à 3, dans lequel un écartement (W) entre les deux bords latéraux (24) du corps de base (18) est constant le long du corps de base (18), l'écartement étant de préférence compris entre 3,0 mm et 10,0 mm, avantageusement entre 7,0 mm et 9,0 mm.

**5.** Outil (10A, 10B) selon l'une quelconque des revendications 1 à 4, dans lequel chaque sillon (22) présente un fond (32) formé par le corps de base (18) et l'ensemble des fonds (32) des sillons (22) suivent une forme ayant un rayon de

courbure principale (r) supérieur ou égal à 2,0 cm, en projection dans un plan parallèle à l'axe longitudinal (A) et perpendiculaire à l'axe transversal (Y).

**6.** Outil (10A, 10B) selon la revendication 5, dans lequel le rayon de courbure principale (r) est compris entre 1 cm et 10 cm de sorte que l'ensemble des fonds (32) des sillons (22) suivent une forme bombée, ou le rayon de courbure principale (r) est de sorte que l'ensemble des fonds (32) des sillons (22) suivent une forme plane.

**7.** Outil (10A, 10B) selon la revendication 6, dans lequel l'organe de mise en forme (14) présente au moins un plan de symétrie perpendiculaire à l'axe longitudinal (A).

**8.** Outil (10A, 10B) selon l'une quelconque des revendications 1 à 7, dans lequel la hauteur (D) de chaque nervure (20) suivant la direction d'élévation (Z) est comprise entre 1,0 mm et 4,0 mm, de préférence comprise entre 2,0 mm à 3,0 mm.

**9.** Outil (10A, 10B) selon l'une quelconque des revendications 1 à 8, dans lequel chaque nervure (20) s'étend à partir du corps de base (18) suivant une direction d'élévation (Z) respective jusqu'à un sommet (36), l'écart (P) entre les sommets (36) de deux nervures (20) adjacentes étant constant et avantageusement compris entre 0,3 mm et 5,0 mm, de préférence compris entre 1,0 mm et 2,0 mm.

**10.** Outil (10A, 10B) selon l'une quelconque des revendications 1 à 9, dans lequel chaque nervure (20) s'étend à partir du corps de base (18) suivant une direction d'élévation (Z) respective jusqu'à un sommet (36), la distance (L) entre les sommets (36) des deux nervures (30A, 30B) les plus éloignées l'une de l'autre étant comprise entre 1,0 cm et 5,0 cm, de préférence comprise entre 2,0 cm et 4,0 cm, avantageusement comprise entre 3,0 cm et 4,0 cm.

**11.** Outil (10A, 10B) selon l'une quelconque des revendications 1 à 10, dans lequel chaque nervure (20) présente une forme en pointe triangulaire, en créneau, ou bombée, en coupe perpendiculaire à l'axe transversal (Y).

**12.** Kit d'application cosmétique comprenant :

- un outil de mise en forme (10A, 10B) selon l'une quelconque des revendications 1 à 11 ;
- une composition cosmétique de mascara ; et
- un applicateur de la composition cosmétique distinct de l'outil de mise en forme (10A, 10B).

**13.** Kit d'application cosmétique comprenant :

- un outil de mise en forme (10A, 10B) selon l'une quelconque des revendications 1 à 11;
- une composition comprenant au moins 30% en poids d'eau par rapport au poids total de la composition, formant une phase aqueuse, au moins 1% en poids par rapport au poids total de la composition de cystéine et/ou d'un de ses sels, une phase lamellaire L$\beta$ formée par un système tensioactif structurant la phase aqueuse, et ladite composition ayant un pH supérieur à 8,0 ; et
- un applicateur de la composition distinct de l'outil de mise en forme (10A, 10B).

**14.** Kit d'application cosmétique comprenant :

- un outil de mise en forme (10A, 10B) selon l'une quelconque des revendications 1 à 11 ;
- une première composition comprenant au moins 30% en poids d'eau par rapport au poids total de la composition, formant une phase aqueuse, au moins 1% en poids par rapport au poids total de la composition de cystéine et/ou d'un de ses sels, une phase lamellaire L$\beta$ formée par un système tensioactif structurant la phase aqueuse, et ladite composition ayant un pH supérieur à 8,0 ;
- un applicateur de la première composition distinct de l'outil de mise en forme (10A, 10B) ;
- une deuxième composition cosmétique de mascara ; et
- un applicateur de la deuxième composition cosmétique distinct de l'outil de mise en forme (10A, 10B) et distinct de l'applicateur de la première composition.

**15.** Procédé de mise en forme de fibres kératiniques comprenant :

- la fourniture d'un outil de mise en forme (10A, 10B) selon l'une quelconque des revendications 1 à 11 ;
- une étape préliminaire d'application d'une composition sur les fibres kératiniques par un applicateur propre,

l'applicateur propre étant distinct de l'outil de mise en forme (10A, 10B) ;
- le recourbement des fibres kératiniques par application de l'outil de mise en forme (10A, 10B) sur les fibres.

**16.** Procédé de maquillage des fibres kératiniques, notamment des cils, comprenant une première étape d'application sur lesdites fibres kératiniques d'une composition comprenant :

au moins 30% en poids d'eau par rapport au poids total de la composition, formant une phase aqueuse,
au moins 1% en poids par rapport au poids total de la composition de cystéine et/ou d'un de ses sels,
une phase lamellaire Lβ formée par un système tensioactif structurant la phase aqueuse,
et ladite composition ayant un pH supérieur à 8,0,

puis une seconde étape de mise en forme desdites fibres par application sur ces dernières d'un outil de mise en forme (10A, 10B), ledit outil comprenant un organe de préhension (12) s'étendant suivant un axe longitudinal (X), et un organe de mise en forme (14) comprenant un corps de base (18) et des nervures (20), le corps de base (18) étant solidaire de l'organe de préhension (12), et présentant deux bords latéraux (24), et s'étendant depuis l'organe de préhension (12) jusqu'à un bord distal libre (26) opposé à l'organe de préhension (12), le bord distal libre (26) joignant les deux bords latéraux (24) l'un à l'autre,
chaque nervure (20) s'étendant en saillie à partir du corps de base (18) suivant une direction d'élévation (Z) respective, les nervures (20) s'étendant aussi parallèlement les unes aux autres et à un axe transversal (Y) et définissant entre elles des sillons (22).

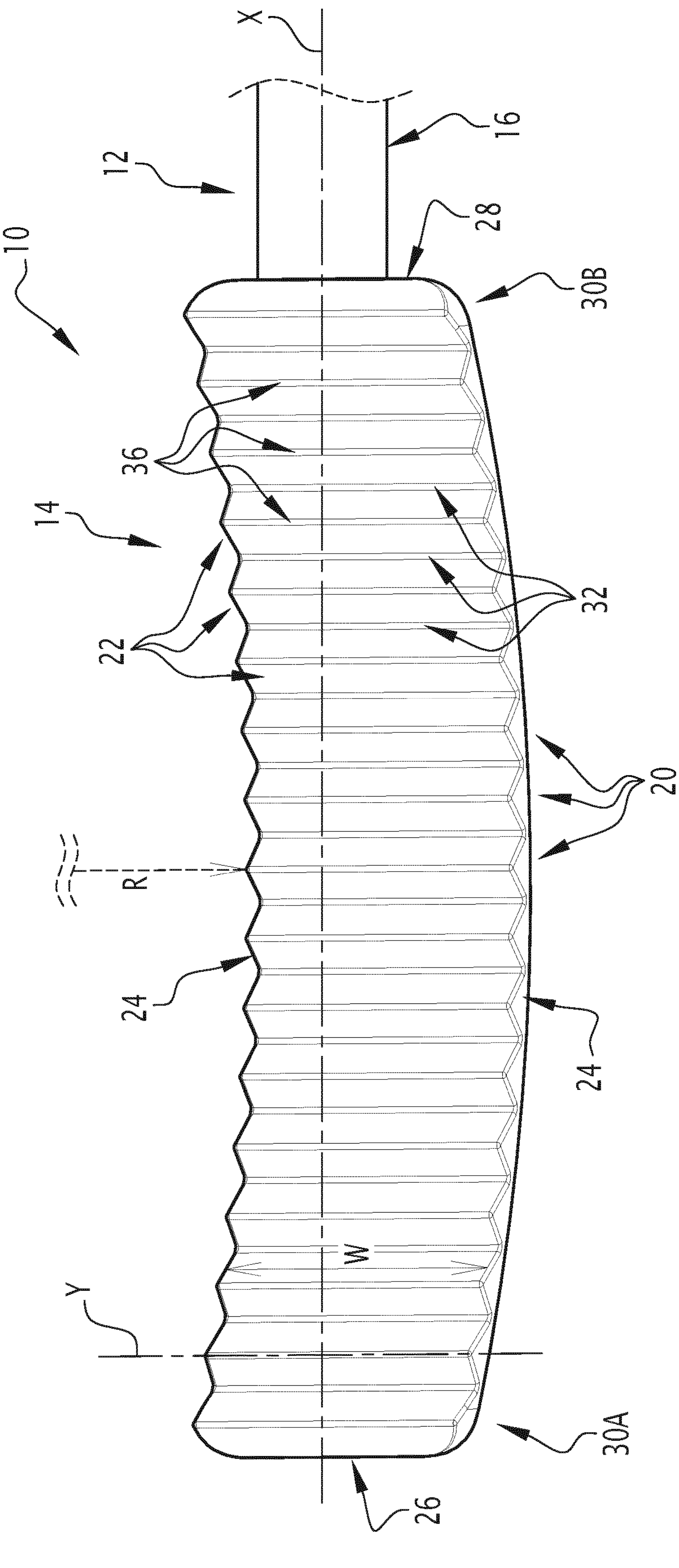


FIG.1

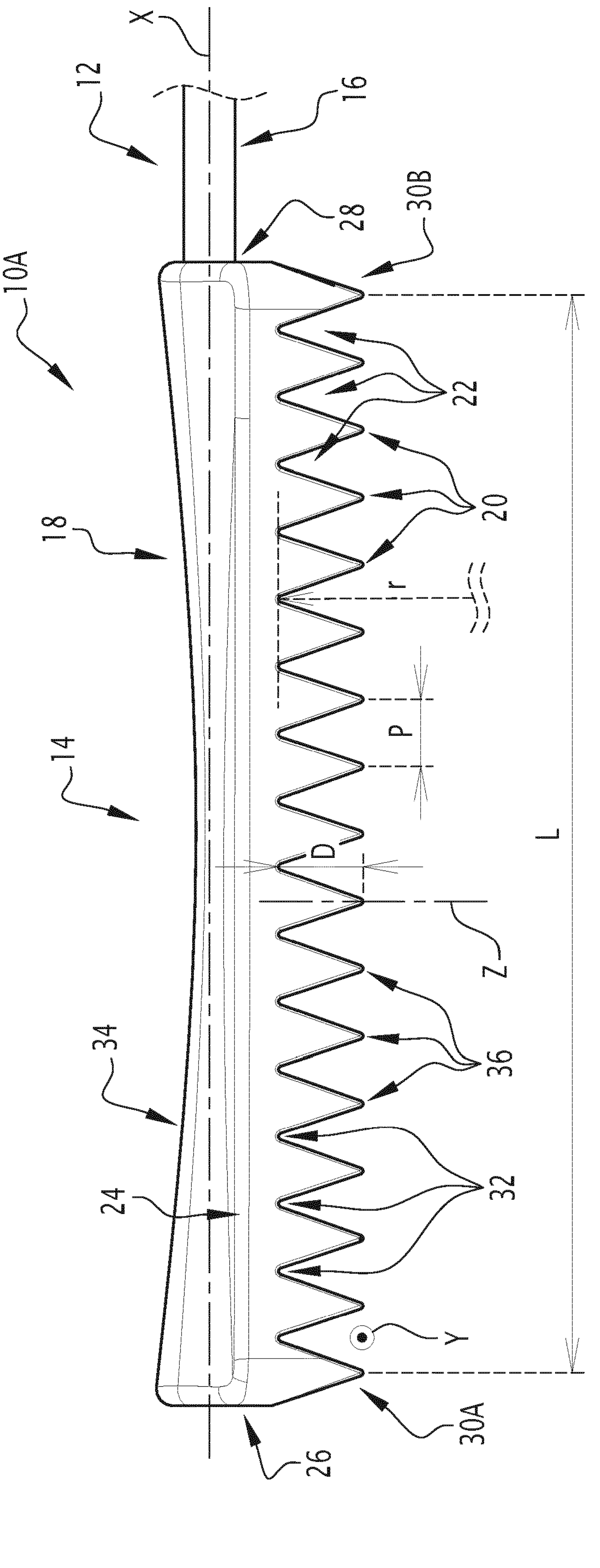


FIG.2

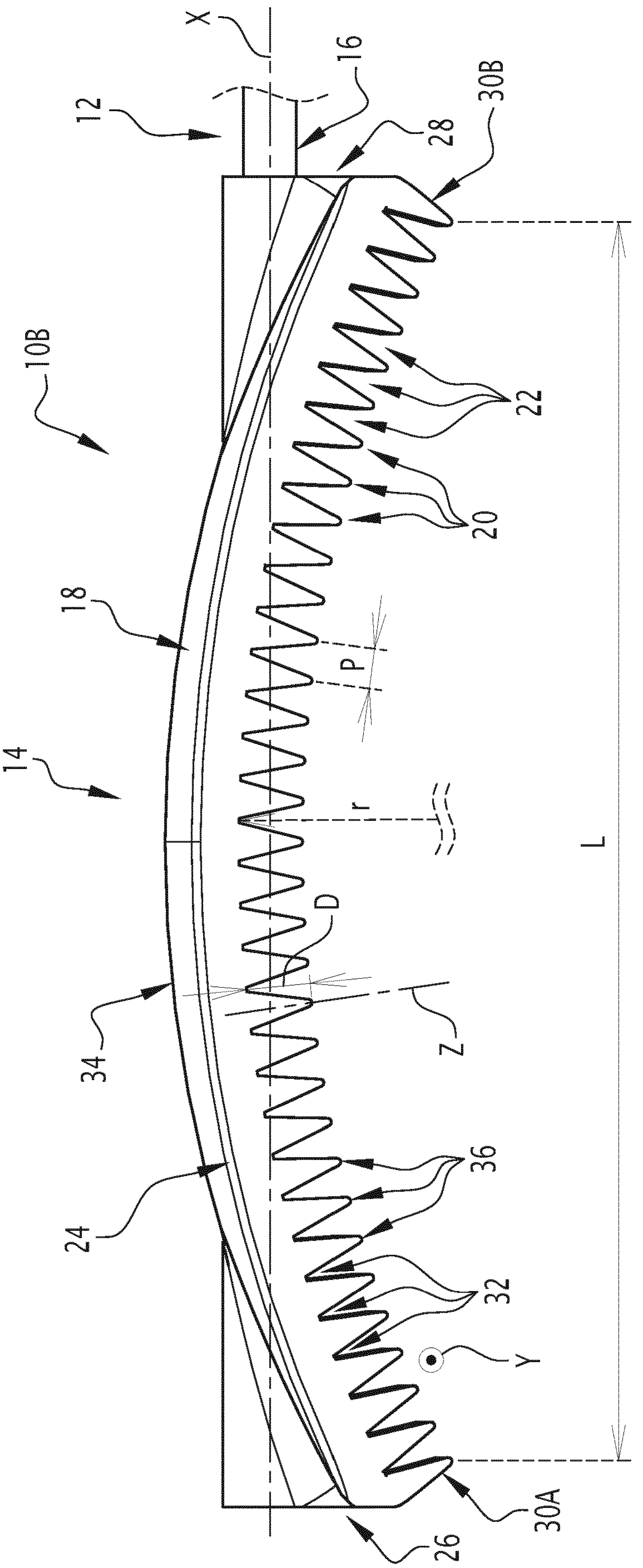


FIG.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005192996 A
- JP 61199807 B
- KR 101823336
- KR 101823401
- FR 591
- FR 2792190 A


### Non-patent literature cited in the description


- *J. Soc. Cosm. Chem.*, 1954, vol. 5, 249-256
- **KIRK-OTHMER**. Encyclopedia of Chemical Technology. WILEY, 1979, vol. 22, 333-432, 347-377